(19) **Europäisches Patentamt / European Patent Office / Office européen des brevets**

(11) **EP 3 359 607 B1**

(12) # FASCICULE DE BREVET EUROPEEN

(45) Date de publication et mention de la délivrance du brevet:
**25.03.2020 Bulletin 2020/13**

(21) Numéro de dépôt: **16788178.8**

(22) Date de dépôt: **06.10.2016**

(51) Int Cl.:
**C09D 4/06** *(2006.01)*    **C08F 220/18** *(2006.01)*

(86) Numéro de dépôt international:
**PCT/FR2016/052583**

(87) Numéro de publication internationale:
**WO 2017/060638 (13.04.2017 Gazette 2017/15)**

(54) **OLIGOMERE IONIQUE ET COMPOSITION POLYMERISABLE LE CONTENANT POUR MATERIAUX HYDRO-FRAGMENTABLES A USAGE PROVISOIRE**

IONISCHES OLIGOMER UND POLYMERISIERBARE ZUSAMMENSETZUNG DAMIT ZUR TEMPORÄREN VERWENDUNG WASSERFRAGMENTIERBARER MATERIALIEN

IONIC OLIGOMER, AND POLYMERIZABLE COMPOSITION CONTAINING SAME FOR TEMPORARY-USE WATER-FRAGMENTABLE MATERIALS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.10.2015 FR 1559638**

(43) Date de publication de la demande:
**15.08.2018 Bulletin 2018/33**

(73) Titulaire: **Arkema France
92700 Colombes (FR)**

(72) Inventeurs:
- **MONNIER, Guillaume
60190 Avrigny (FR)**
- **DUQUENNE, Christophe
75010 Paris (FR)**
- **BEAUDRAIS, Sylvain
60600 Agnetz (FR)**

(74) Mandataire: **Schaefer, Anne-Sophie et al
Arkema France
Département Propriété Industrielle
420, rue d'Estienne d'Orves
92705 Colombes Cedex (FR)**

(56) Documents cités:
CA-A1- 2 239 439    US-A- 5 030 747
US-A- 5 792 827    US-A1- 2012 157 351
US-A1- 2013 090 444

**EP 3 359 607 B1**

## Description

La présente invention selon les revendications 1 à 18 concerne un oligomère spécifique porteur d'au moins une insaturation éthylénique réticulable et d'au moins une liaison ionique sous forme de carboxylate d'ammonium, lequel oligomère comprend dans sa structure au moins un groupement aminoacrylate et au moins une amine tertiaire y compris aminoacrylate, sous forme salifiée de carboxylate d'ammonium par au moins un composé acide carboxylique. Plus particulièrement, l'oligomère, objet de la présente invention, résulte de l'association par liaison ionique carboxylate d'ammonium entre un oligomère précurseur porteur dudit au moins un groupement aminoacrylate et dudit composé acide carboxylique, lequel composé acide peut être un monoacide ou un polyacide et en particulier un polyacide qui est un diacide, ledit composé acide pouvant être saturé ou insaturé et ledit oligomère précurseur pouvant être porteur d'insaturation éthylénique polymérisable, en particulier acrylate ou pouvant être sans aucune insaturation éthylénique avec, dans ce cas, l'insaturation éthylénique de l'oligomère de la présente invention étant apportée par ledit composé acide carboxylique.

L'oligomère selon l'invention a comme objectif principal son utilisation dans des applications concernant des matériaux à fonction ou usage provisoire comme des revêtements ou des objets pouvant être éliminés facilement après une fonction provisoire, par simple nettoyage à l'eau ou l'eau saline ou une autre solution aqueuse, en particulier ayant un pH > 7 et de préférence > 8, plus particulièrement par un jet d'eau et en option à une température adaptée et supérieure à la température de transition vitreuse du produit réticulé obtenu. Un avantage particulier recherché de ces revêtements et articles ou pièces fonctionnels est qu'ils sont respectueux de la santé des opérateurs et de l'environnement en général sans utilisation de solvants dangereux ou de produits corrosifs et pouvant affecter la santé et l'environnement. Des applications plus particulières visées sont également liées à leur forte sensibilité à l'eau et leur capacité de former des gels dans l'eau (hydrogels) pouvant servir comme vecteurs de principes actifs divers en milieu aqueux et facilement éliminables si nécessaire.

Plus particulièrement, la présente invention concerne des produits réticulés à partir desdits oligomères selon l'invention, avec des produits réticulés qui sont hydrofragmentables ou hydrosolubles permettant ainsi leur élimination complète par l'eau ou en milieu aqueux comme exposé ci-haut. En particulier, ce type de produit a un intérêt dans une composition réticulable pour servir de matériau support (dit aussi matériau sacrificiel) aux pièces en cours d'élévation dans la technique d'impression 3D, en particulier selon la technique Inkjet 3D/polyjet avec projection de composition réticulable (dite aussi résine) et cuisson sous rayonnement, en particulier UV couche après couche. Ledit support ou matériau sacrificiel est par la suite éliminé à l'aide d'un simple passage dans un bain d'eau avec le pH et la température du bain étant adaptée à la technique et à la composition réticulable utilisée. Dans certains cas, il peut être nécessaire d'avoir une agitation ou un temps de trempage et/ou présence d'additifs permettant cette élimination.

Avant réticulation sous rayonnement et en particulier sous lampe UV, la composition réticulable comprenant ledit oligomère réticulable doit présenter une viscosité inférieure à 30 mPa.s à la température d'éjection de ladite composition laquelle est typiquement supérieure à 45°C. Durant le processus de fabrication, le matériau sacrificiel doit apporter les caractéristiques attendues d'un support avec essentiellement une bonne résistance mécanique pour satisfaire à la fonction de matériau de « support » et une vitesse minimale de cuisson (réticulation) sous rayonnement, en particulier UV. De préférence, la réactivité correspond à une vitesse de cuisson (ou de réticulation) équivalente à moins de 1 passage à 10 m/min sous lampe UV de 120 Watt/cm pour un revêtement 25 microns. Une bonne stabilité dimensionnelle avec un faible retrait, de préférence < 1% peut être également d'intérêt.

Au terme de l'impression tridimensionnelle, le matériau sacrificiel (support) doit se dissoudre ou être éliminé par hydro-fragmentation rapidement dans l'eau ou une solution aqueuse sans laisser de marques sur la surface de l'objet 3D ainsi produit. Le matériau à usage provisoire à sacrifier est hydro-fragmentable selon la présente invention si, après mise dans l'eau ou dans une solution aqueuse saline ou basique (pH > 7) sous agitation magnétique, le matériau se désintègre sous forme d'une fraction « hydrodispersible » ou « hydrosoluble » (passant dans la phase aqueuse après filtration sur papier filtre standard) et/ou une fraction solide résiduelle (après filtration) de taille de particules homogènes et ne dépassant pas 10 mm. Plus préférentiellement, de cette désintégration résultent deux fractions comme définies ci-haut. Comme test convenable pour caractériser le degré d'hydro-fragmentabilté, on peut utiliser le test tel que décrit dans la partie expérimentale de la description.

Le matériau support sacrificiel entourant l'objet 3D visé a comme fonction provisoire de soutenir progressivement la forme dudit article au fur et à mesure de la progression du nombre de couches imprimées et réticulées une à une.

De l'état de la technique, les solutions connues les plus courantes de compositions réticulables pouvant servir de matériau « support » de l'objet 3D imprimé couche par couche sont, soit à base de matériau inerte (non réactif sous rayonnement) et hydrosoluble, soit à base d'une composition polymérisable à base de monomères ou d'oligomères mono éthyléniques hydrosolubles polymérisables sous rayonnement et comprenant comme composant essentiel des composants oligomères ou polymères inertes hydrosolubles ou autres additifs hydrosolubles rendant le matériau « support » éliminable par dissolution dans l'eau.

Par exemple, US 2013/0234370 décrit une composition pour matériau support hydrosoluble comprenant un

2

**EP 3 359 607 B1**

monomère polymérisable monofonctionnel hydrosoluble et un polyoxypropylène glycol de Mn allant de 100 à 5000 et/ou de l'eau.

US 2013/0337277 décrit une composition liquide pour structures temporaires auto-destructibles pour un procédé d'impression 3D avec ladite composition comprenant un copolymère biodégradable par dégradation enzymatique avec activation d'une enzyme biospécifique après finition de l'objet 3D. Ledit copolymère peut comprendre du polyéthylène glycol, de l'acide polyacrylique, de l'acide polyhyaluronique, de la polycaprolactone ou du polyvinyl alcool.

US 2012/0178845 décrit également des compositions réticulables sous rayonnement pour impression 3D sous rayonnement et, en particulier, une composition convenable comme support de l'objet 3D ayant comme composant réactif un composant acrylique ou composant porteur de vinyl éthers ou un composant miscible à l'eau, lequel gonfle dans l'eau ou dans une solution basique ou acide après réticulation, ledit composant pouvant être un oligomère uréthane acrylé à base de polyéthylène glycol ou un oligomère polyol partiellement acrylé ou un oligomère acrylé ayant des substituants hydrophiles parmi amine, hydroxy ou acide. Un composant non réactif est également présent choisi parmi le polyéthylène glycol (PEG), méthoxy PEG, glycérol, polyol polyéthoxylé ou du propylène glycol.

CA 2,239,439 concerne d'autres applications que l'impression d'objets 3D et décrit un procédé de production de peintures aqueuses réticulables sous rayonnement, lesquelles peintures sont solubles dans l'eau et obtenues par mélange d'une pré-peinture A à base d'un liant oligomère ou polymère, porteur d'au moins deux insaturations éthyléniques qui n'est pas miscible à l'eau, avec une amine B) primaire-tertiaire à 0,2 à 5% en poids par rapport à ladite pré-peinture avec incorporation de ladite amine dans ledit oligomère ou polymère, suivi de la neutralisation des groupements amino par un acide et solubilisation dans l'eau de la peinture et ajustement de sa viscosité. Aucune mention d'utilisation d'une telle composition une fois réticulée comme matériau à fonction provisoire et temporaire et en particulier éliminable par l'eau n'est décrite ou enseignée.

CA 2,239,310 décrit des compositions similaires au cas précédent, des peintures aqueuses, mais en partant d'un liant réactif compatible, dispersible ou soluble dans l'eau et avec augmentation de cette compatibilité (solubilité) par modification dudit liant avec une amine et neutralisation par un acide pour améliorer la solubilité du liant de la peinture avant réticulation sous rayonnement.

US 5 792 827 décrit des compositions aqueuses de revêtements pour bois ou papier, réticulables sous rayonnement et à base d'un adduit d'un acrylate multifonctionnel avec une amine. Les amines peuvent être neutralisées par un acide comme l'acide lactique, acétique, formique ou phosphorique.

US 2012/0157351 décrit une méthode d'inhibition de la formation d'agglomérats hydratés dans un fluide parmi l'eau, le gaz et en option un hydrocarbure liquide, comprenant l'addition dans ledit fluide d'un additif anti-agglomérant ou de ses sels. Comme produits additifs, sont exemplifiés des produits d'addition de la diméthyl propylamine ou de dibutyl propylamine sur le 2-éthyl hexyl acrylate avec salification de l'amine tertiaire par respectivement le 1-chloro butane ou par l'acide acétique. Aucune utilisation dans une composition polymérisable ou réticulable et aucune utilisation pour des matériaux à fonction provisoire n'est décrite, ni suggérée.

Aucun des documents cités ne décrit, ni n'enseigne le principe de la présente invention pour l'obtention des matériaux à fonction provisoire et facilement éliminables par l'eau et suivant les besoins, soit comme support d'objets imprimés en 3D couche par couche (sous rayonnement), soit comme revêtements provisoires ou comme vecteur de principe actif provisoire, également éliminable à l'eau et en particulier le principe d'un édifice réticulé mixte avec des liaisons ioniques réversibles et associant des structures hydrophiles ayant une viscosité adaptée à l'application et une tenue mécanique suffisante pour remplir leur fonction d'usage comme matériau provisoire éliminable à l'eau ou par une solution aqueuse adaptée.

En particulier, la composition de l'oligomère selon l'invention ou la composition polymérisable et en particulier réticulable le comprenant, plus particulièrement conduisant par réticulation au matériau support d'objet 3D, est exempte de tout composant libre ou d'additif libre hydrosoluble et non réactif par réticulation comme décrits dans l'état de la technique cité ci-haut.

L'invention concerne d'abord un oligomère qui est ionique en étant porteur d'au moins une liaison ionique carboxylate d'ammonium et étant obtenu à partir d'un oligomère précurseur P comprenant au moins un groupement aminoacrylate et d'un composé acide salifiant par ladite liaison ionique carboxylate d'ammonium, au moins une amine tertiaire dudit oligomère P, laquelle peut être un aminoacrylate ou en option un autre groupement amine tertiaire (groupement ou fonction amine ayant la même signification pour la suite) porté par un composé amine formant ledit au moins un groupement aminoacrylate.

L'invention concerne également ledit oligomère précurseur P en tant qu'intermédiaire de préparation dudit oligomère tel que défini selon la présente invention.

Fait également partie de l'invention, selon la revendication 19, une solution dudit oligomère dans un diluant réactif et une composition polymérisable, en particulier réticulable, comprenant au moins un oligomère tel que défini selon l'invention.

Un autre objet de l'invention selon la revendication 20 concerne un procédé de préparation dudit oligomère comprenant d'abord la préparation d'un oligomère précurseur P et ensuite sa salification par un composé acide car-

3

boxylique sous forme de carboxylate d'ammonium d'au moins une amine tertiaire dudit oligomère P, pouvant être au moins un desdits groupements aminoacrylates et en option au moins une amine tertiaire portée par l'amine d'addition de départ formant ledit groupement aminoacrylate.

Fait également partie de la présente invention, selon les revendications de 23 à 26 l'utilisation dudit oligomère ou de sa solution dans un diluant réactif ou d'une composition polymérisable, en particulier réticulable le comprenant ou de l'oligomère tel qu'obtenu par le procédé décrit selon l'invention, (l'utilisation) en tant que liant polymérisable et plus particulièrement réticulable pour des applications dans les matériaux à fonction ou usage provisoire, éliminables par l'eau, par une solution aqueuse saline ou par une autre solution aqueuse qui est basique, de préférence pour revêtements, hydrogels ou pour matériau support pour objet d'impression en 3D couche par couche.

Finalement, la présente invention selon la revendication 27 couvre également le produit fini matériau polymère, en particulier réticulé, obtenu par polymérisation (polymère) et en particulier réticulation (produit réticulé) d'une composition comprenant ledit oligomère ionique.

Donc, le premier objet de l'invention concerne un oligomère polymérisable en particulier réticulable, porteur d'au moins une insaturation éthylénique et d'au moins une liaison ionique de carboxylate d'ammonium et comprenant dans sa structure ou dans sa composition :

P) au moins un oligomère précurseur, porteur d'au moins une fonction amine tertiaire sous forme de groupement aminoacrylate, de préférence d'au moins deux fonctions amines tertiaires sous forme de groupements aminoacrylates, résultant de l'addition de A) au moins un composé amine selon A1) porteur d'au moins une fonction amine primaire ($-NH_2$) et/ou secondaire (-NH) et en option d'au moins une fonction amine tertiaire et/ou selon A2) porteur d'au moins une fonction amine secondaire (-NH) et en option d'au moins une fonction amine tertiaire (avec le composé amine selon A2) n'ayant aucune fonction amine primaire-$NH_2$), sur B) au moins un composé acrylate hydrophile avec formation ainsi dudit au moins un groupement aminoacrylate, et

C) au moins un composé acide carboxylique attaché audit oligomère précurseur P par au moins une liaison ionique de carboxylate d'ammonium avec au moins un desdits groupements aminoacrylates et en option (avec) au moins une desdites fonctions amines tertiaires dudit composé amine A),

de préférence ledit oligomère étant porteur d'au moins deux liaisons ioniques carboxylates d'ammonium telles que définies ci-avant.

Le terme « polymérisable » signifie « pouvant polymériser par le port d'au moins une insaturation éthylénique polymérisable » et le terme « réticulable » est un cas plus spécifique du terme « polymérisable » où ledit oligomère « peut réticuler déjà seul et donc porte au moins 2 insaturations éthyléniques polymérisables par molécule ». Il est évident que ledit oligomère s'il est « polymérisable » et « réticulable » seul est également et respectivement « copolymérisable » et « coréticulable » dans une composition de monomères et/ou d'autres oligomères « polymérisables» ou « réticulables ».

Un acrylate « hydrophile » signifie selon la présente invention que ledit acrylate est soluble dans l'eau ou hydrodispersible dans l'eau sans tensioactif dit aussi « auto dispersible » dans l'eau.

Selon une première option particulière dans ledit oligomère de l'invention, ledit composé amine A) peut être sélectionné parmi :

A1) un composé amine, porteur d'au moins une, en particulier une fonction amine primaire et en option, d'au moins une, en particulier une fonction amine tertiaire et/ou d'au moins une, en particulier une fonction amine secondaire et/ou

A2) un composé amine porteur d'au moins une, en particulier une fonction amine secondaire et en option d'au moins une, en particulier une fonction amine tertiaire,

et avec ledit composé acrylate B) étant au moins un composé acrylate multifonctionnel selon B1) et/ou monofonctionnel selon B2) et en particulier dans le cas où A) est un composé selon A1) ou comprend un composé amine selon A1), ledit composé acrylate B) est un mélange d'au moins un acrylate multifonctionnel selon B1) et d'au moins un acrylate monofonctionnel selon B2).

Plus particulièrement, ledit composé acrylate hydrophile B) est un mélange de composé acrylate (hydrophile) multifonctionnel selon B1) et de composé acrylate (hydrophile) monofonctionnel selon B2).

Ledit groupement aminoacrylate de l'oligomère de l'invention peut porter au moins une insaturation éthylénique, en particulier acrylate ou aucune insaturation éthylénique et dans ce dernier cas, ledit composé acide C) est éthyléniquement insaturé.

Selon une option préférée dudit oligomère, ledit composé acide C) est un monoacide selon C1) qui est éthyléniquement insaturé selon C11) et ledit oligomère porte au moins deux liaisons ioniques carboxylates d'ammonium.

Différentes options existent pour ledit composé acide carboxylique C). D'abord :

- quand ledit composé acide carboxylique C) est selon C1) un monoacide, dans ce cas :

   C1) peut être un monoacide selon C11) qui est éthyléniquement insaturé et au moins une insaturation éthylénique dudit oligomère est portée par au moins ledit monoacide selon C11) par liaison carboxylate d'ammonium avec au moins une desdites fonctions amines tertiaires parmi lesdits groupements aminoacrylates ou en option parmi les fonctions amines tertiaires dudit composé amine A) et éventuellement au moins une autre insaturation éthylénique dudit oligomère est portée par au moins un groupement aminoacrylate sous forme de groupement aminoacrylate-acrylate terminal dudit oligomère précurseur P ou

   C1) peut être un monoacide selon C12) qui est saturé et au moins une insaturation éthylénique dudit oligomère est portée par au moins un groupement aminoacrylate sous forme de groupement aminoacrylate-acrylate terminal dudit oligomère précurseur P, de préférence ledit monoacide selon C1) étant selon C11) un monoacide insaturé et

- quand ledit composé acide carboxylique C) est selon C2) un polyacide carboxylique et de préférence un diacide, dans ce cas :

   C2) peut être un polyacide selon C21) qui est éthyléniquement insaturé, en particulier diacide éthyléniquement insaturé et au moins une insaturation éthylénique dudit oligomère est portée par au moins ledit polyacide selon C21) et éventuellement en plus, au moins une insaturation éthylénique est portée par au moins un groupement aminoacrylate sous forme de groupements aminoacrylate-acrylate terminal dudit oligomère précurseur P ou

   C2) peut être un polyacide selon C22) saturé, en particulier diacide saturé et ledit oligomère précurseur P porte au moins un groupement aminoacrylate-acrylate terminal et ledit oligomère qui résulte porte au moins deux insaturations éthyléniques acrylates.

Ledit groupement « aminoacrylate » correspond au groupement de liaison formé lors de la réaction d'addition d'un groupement « =NH » dudit composé amine A) sur un groupement acrylate $CH_2=CH-CO_2$-, avec ledit groupement aminoacrylate représenté par la formule suivante :

$$\text{« } =N-CH_2CH_2-CO_2\text{- »} \qquad (1)$$

Un groupement « aminoacrylate-acrylate terminal » signifie un groupement acrylate résiduel d'un acrylate multifonctionnel selon B1), après formation d'un groupement « aminoacrylate » comme expliqué ci-dessus avec un groupement acrylate d'un acrylate selon B1) : $(CH_2=CH-CO_2)_n-R$ de fonctionnalité n en acrylates d'au moins 2, selon schéma suivant :

$$=NH + (CH_2=CH-CO_2)_n-R \rightarrow =N-CH_2CH_2-CO_2-R(-O_2C-CH=CH_2)_{n-1} \qquad (2)$$

Dans le cas ci-dessus, pour une addition =NH sur un groupement acrylate, il y a n-1 groupements acrylates portés par ledit groupement « aminoacrylate » et donc n-1 groupements « aminoacrylate-acrylates terminaux ».

Selon une option préférée, ledit composé acide carboxylique C) dans ledit oligomère selon l'invention est un diacide insaturé selon C21) ou saturé selon C22) associant par deux liaisons ioniques carboxylates, deux molécules dudit oligomère précurseur P, par salification sous forme de sel de carboxylate d'ammonium d'une desdites fonctions amines tertiaires parmi lesdits groupements aminoacrylates formés ou en option parmi lesdites fonctions amines tertiaires dudit composé amine A), sur chacune desdites molécules dudit oligomère P ou ledit composé acide carboxylique C) est un monoacide selon C1), éthyléniquement insaturé selon C11) ayant une insaturation éthylénique polymérisable et ledit monoacide selon C11) salifie au moins deux desdites fonctions amines tertiaires sous forme de (sel de ou de liaison ionique de) carboxylate d'ammonium.

Plus particulièrement, dans le cas d'un diacide selon C21) ou selon C22), on peut schématiser ledit oligomère selon l'invention par la formule générale (3) suivante :

$$P(NH^+)_l[^-O_2C-R'-CO_2^-]_m(^+HN)_l P \qquad (3)$$

avec R' étant éthyléniquement insaturé si ledit composé acide C) est selon C21) et R' étant saturé si ledit composé acide C) est selon C22).

« l » étant le nombre de sites carboxylates d'ammonium par molécule d'oligomère précurseur P et « m » le nombre de molécules de diacide entre deux chaînes (molécules) de précurseur P avec l = m.

Plus particulièrement, dans l'oligomère selon la présente invention, ledit oligomère précurseur P comprend dans sa structure, des segments de chaîne hydrophiles qui peuvent être hydrosolubles (solubles dans l'eau) ou hydrodispersibles (dispersibles dans l'eau sans addition de tensioactif), en particulier sélectionnés parmi les polyéthers ou parmi les polyesters, en particulier polyesters à base d'oligoéthers polyols ou de polyols éthoxylés, ou parmi les polyuréthanes à base d'oligoéthers polyols ou de polyols éthoxylés, en particulier parmi les polyuréthanes à base d'oligoéther polyols, en particulier lesdits segments ayant une masse moléculaire moyenne en nombre Mn inférieure à 2000, de préférence inférieure à 1000. La Mn est en particulier calculée à partir de l'indice OH, $I_{OH}$, du polyol ou monool exprimé en mg KOH/g et de sa fonctionnalité f en hydroxy. La Mn est calculée à partir de :

$$Mn = f*56000 / I_{OH}$$

La structure hydrophile préférée pour ledit oligomère précurseur P sont des polyéthers ou oligoéthers à base de polyoxyéthylène de Mn comme indiquée ci-dessus.

Selon une première option plus particulière dudit oligomère de l'invention, en fonction du composé acide carboxylique C) choisi, ledit composé acide carboxylique C) est selon C11) un monoacide éthyléniquement insaturé et sélectionné parmi : acide acrylique ou méthacrylique, acide crotonique (acide trans-2-butènoïque) ou le β-carboxyéthyl acrylate (β-CEA) ou leurs mélanges, de préférence l'acide acrylique ou méthacrylique ou β-CEA ou leurs mélanges et plus préférentiellement l'acide acrylique ou méthacrylique ou leurs mélanges.

Selon une autre option plus particulière, ledit composé acide carboxylique C) est selon C22) un diacide carboxylique saturé et sélectionné parmi : acide succinique, acide malonique, acide malique, acide glutarique (en $C_5$ : porteur de 5 atomes de carbone), acide adipique (en $C_6$), acide pimélique (en $C_7$) ou diester-acides de diacides précités avec un alcane diol en $C_2$ à $C_4$ ou avec un di-, tri- ou tetra-éthylène glycol ou diacides parmi les dimères et/ou trimères d'acides gras, en particulier dimères et/ou trimères d'acides gras hydrogénés (dimères en $C_{36}$ et trimères en $C_{54}$) ou leurs mélanges, en particulier par deux ou par trois et de préférence parmi l'acide succinique, acide malonique, acide malique, acide glutarique (en $C_5$) ou diacides parmi les dimères et/ou trimères d'acides gras, en particulier dimères et/ou trimères d'acides gras hydrogénés ou leurs mélanges.

Selon une troisième option plus particulière, ledit composé acide carboxylique C) est selon C21) un diacide carboxylique insaturé et sélectionné parmi : acide itaconique, acide maléique, acide fumarique, acide tetrahydrophtalique (acide cyclohexène dioïque), ou diesters acides des diacides précités avec un alcane diol en $C_2$ à $C_4$ ou avec un di-, tri- ou tetra-éthylène glycol ou les dimères et/ou trimères d'acides gras non hydrogénés (dimères en $C_{36}$ et trimères en $C_{54}$) ou leurs mélanges, de préférence parmi l'acide itaconique, acide maléique, acide fumarique et leurs mélanges.

La structure dudit oligomère de l'invention et en particulier l'insaturation éthylénique et sa position dépendra de la présence ou absence d'une telle insaturation dans ledit oligomère précurseur P et en fonction de la présence ou absence d'une telle insaturation éthylénique dans ledit composé acide carboxylique C) suivant qu'il est saturé ou insaturé.

Ledit oligomère précurseur P soit il ne porte aucun groupement aminoacrylate-acrylate terminal (aucun acrylate), soit il porte au moins un groupement aminoacrylate-acrylate terminal (un acrylate) et que :

    a) dans le cas où ledit oligomère précurseur P porte un groupement aminoacrylate-acrylate terminal :

        a1) ledit composé acide carboxylique C) est un monoacide éthyléniquement insaturé selon C11) ou
        a2) ledit composé acide carboxylique C) est un diacide insaturé selon C21) ou un diacide saturé selon C22), de préférence saturé selon C22),

    b) dans le cas où ledit oligomère précurseur P porte aucun groupement aminoacrylate-acrylate terminal, dans ce cas :

        b1) ledit composé acide carboxylique C) est un monoacide insaturé selon C11) et deux molécules dudit monoacide selon C11) sont attachées audit oligomère précurseur P, par deux liaisons ioniques ou
        b2) ledit composé acide carboxylique C) est un diacide insaturé selon C21) ou saturé selon C22), de préférence saturé selon C22) et en présence d'un monoacide insaturé selon C11) ou
        b3) ledit composé acide carboxylique C) est un diacide insaturé selon C21).

La fonctionnalité de l'oligomère de l'invention en insaturations éthyléniques dépendra également de la présence ou de l'absence d'insaturation éthylénique dans ledit oligomère précurseur P et ledit acide carboxylique C). Plus particulièrement, l'oligomère selon l'invention peut avoir une fonctionnalité f en insaturation éthylénique comme suit :

    a') f = 1 avec ledit composé acide carboxylique C) étant selon C11) un monoacide insaturé salifiant (sous forme de sel de carboxylate d'ammonium), une seule desdites fonctions amines tertiaires pouvant être un aminoacrylate formé dans ledit oligomère précurseur P ou en option des fonctions amines tertiaires portées par ledit composé amine A) de départ, ledit oligomère précurseur P ne portant aucun groupement aminoacrylate-acrylate terminal (par molécule) ou

b') f = 2 avec :

- ledit composé acide carboxylique C) étant selon C11) un monoacide insaturé salifiant deux desdites fonctions amines tertiaires pouvant être des aminoacrylates formés dans ledit oligomère précurseur P et/ou en option des fonctions amines tertiaires portées par ledit composé amine A) de départ, ledit oligomère précurseur P ne portant aucun groupement aminoacrylate-acrylate terminal (par molécule) ou
- ledit composé acide carboxylique C) étant selon C21) un diacide saturé salifiant une desdites fonctions amines tertiaires pouvant être des aminoacrylates formés dans ledit oligomère précurseur P ou en option des fonctions amines tertiaires portées par ledit composé amine A) de départ et avec deux liaisons carboxylates d'ammonium liant ledit diacide selon C21) à deux molécules dudit oligomère précurseur P, ledit oligomère précurseur P étant porteur d'un seul groupement aminoacrylate-acrylate terminal (par molécule) ou
- ledit composé acide carboxylique C) étant selon C11) un monoacide insaturé salifiant une desdites fonctions amines tertiaires pouvant être des aminoacrylates formés dans ledit oligomère précurseur P ou en option des fonctions amines tertiaires portées par ledit composé amine A) de départ et ledit oligomère précurseur P étant porteur d'un seul groupement aminoacylate-acrylate terminal,

c') f = 3 avec :

- ledit composé acide carboxylique C) étant selon C11) un monoacide insaturé salifiant trois desdites fonctions amines tertiaires pouvant être des aminoacrylates formés dans ledit oligomère précurseur P et/ou en option desdites fonctions amines tertiaires portées par ledit composé amine A) de départ et avec ledit oligomère précurseur P ne portant aucun groupement aminoacrylate-acrylate terminal ou
- ledit composé acide carboxylique C) étant selon C11) un monoacide insaturé salifiant deux fonctions desdites fonctions amines tertiaires pouvant être des aminoacrylates formés dans ledit oligomère précurseur P et/ou en option des fonctions amines tertiaires portées par ledit composé amine A) de départ, sous forme de carboxylate d'ammonium, avec ledit oligomère précurseur P portant un seul groupement aminoacrylate-acrylate terminal ou
- ledit composé acide C) étant selon C22) un diacide insaturé salifiant une desdites fonctions amines tertiaires pouvant être des aminoacrylates formés dans ledit oligomère précurseur P ou en option des fonctions amines tertiaires portées par ledit composé amine A) de départ, dans chacune de deux molécules d'oligomère précurseur P, ledit oligomère précurseur P étant porteur d'un seul groupement aminoacrylate-acrylate terminal par molécule ou
- ledit composé acide carboxylique C) étant selon C11) un monoacide insaturé salifiant une desdites fonctions amines tertiaires pouvant être des aminoacrylates formés dans ledit oligomère précurseur P ou en option des fonctions amines tertiaires portées par ledit composé amine A) de départ, avec ledit oligomère précurseur P étant porteur de deux groupements aminoacrylate-acrylates terminaux par molécule ou
- ledit composé acide carboxylique C) étant un mélange d'un diacide insaturé selon C22) et d'un monoacide insaturé selon C11), avec ledit diacide selon C22) associant par salification deux molécules dudit oligomère précurseur P ayant chacune desdites molécules, une desdites fonctions amines tertiaires pouvant être des aminoacrylates formés dans ledit oligomère précurseur P ou en option des fonctions amines tertiaires portées par ledit composé amine A) de départ, salifiée par ledit diacide selon C22) et une deuxième desdites fonctions amines tertiaires salifiée par ledit monoacide selon C11), ledit oligomère (final) portant quatre liaisons ioniques carboxylates d'ammonium ou
- ledit composé acide carboxylique C) étant un diacide saturé selon C21) associant par salification deux oligomères précurseurs P dont l'un porte une fonction aminoacrylate-acrylate terminale (par molécule) et l'autre deux fonctions aminoacrylate-acrylates terminales (par molécule)

d') f = 4, avec :

- ledit composé acide carboxylique C) étant un monoacide insaturé selon C11) salifiant quatre desdites fonctions amines tertiaires pouvant être des aminoacrylates formés dans ledit oligomère précurseur P et/ou en option des fonctions amines tertiaires portées par ledit composé amine A) de départ, ledit oligomère P étant porteur d'aucun groupement aminoacrylate-acrylate terminal par molécule ou
- ledit composé acide carboxylique C) étant un diacide saturé selon C21) et associant deux molécules dudit oligomère précurseur P par salification d'une desdites fonctions amines tertiaires pouvant être des aminoacrylates formés dans ledit oligomère précurseur P ou en option des fonctions amines tertiaires portées par ledit composé amine A) de départ, respectivement dans chacune de deux molécules dudit oligomère précurseur P et avec ledit oligomère précurseur P étant porteur de 2 groupements aminoacrylate-acrylates terminaux par

7

molécule ou

- ledit composé acide carboxylique C) étant un diacide saturé selon C21) et associant deux molécules dudit oligomère précurseur P par salification d'une desdites fonctions amines tertiaires pouvant être des aminoacrylates formés dans ledit oligomère précurseur P ou en option des fonctions amines tertiaires portées par ledit composé amine A) de départ, respectivement dans chacune de deux molécules dudit oligomère précurseur P et avec un premier oligomère précurseur P étant porteur de 2 groupements aminoacrylate-acrylates terminaux par molécule et un deuxième étant porteur d'un seul groupement aminoacrylate-acrylate ou

- ledit composé acide carboxylique C) étant un monoacide insaturé selon C11) salifiant trois desdites fonctions amines tertiaires pouvant être des aminoacrylates formés dans ledit oligomère précurseur P et/ou en option des fonctions amines tertiaires portées par ledit composé amine A) de départ, avec ledit oligomère précurseur P étant porteur d'un seul groupement aminoacrylate-acrylate terminal par molécule) ou

- ledit composé acide carboxylique C) étant un monoacide insaturé selon C11) salifiant deux desdites fonctions amines tertiaires pouvant être des aminoacrylates formés dans ledit oligomère précurseur P et/ou en option des fonctions amines tertiaires portées par ledit composé amine A) de départ, avec ledit oligomère précurseur P portant deux groupements aminoacrylate-acrylates terminaux par molécule ou

- ledit composé acide carboxylique C) est un monoacide insaturé selon C11) salifiant une desdites fonctions amines tertiaires pouvant être des aminoacrylates formés dans ledit oligomère précurseur P ou en option des fonctions amines tertiaires portées par ledit composé amine A) de départ, avec ledit oligomère précurseur P portant trois groupements aminoacrylate-acrylates terminaux par molécule,

avec, dans le cas où un diacide selon C21) ou selon C22) est utilisé pour associer par salification deux molécules dudit oligomère précurseur P, lesdites molécules dudit oligomère précurseur P pouvant être de structure identique ou différente.

Selon une option particulière, les composants dudit oligomère précurseur P, c'est-à-dire le composé amine A) et/ou le composé acrylate hydrophile B), peuvent être des mélanges respectifs de composés A) et/ou B). De même, ledit oligomère précurseur P peut être un mélange d'oligomères précurseurs P différents en structure et en fonctionnalité et/ou ledit composé acide carboxylique C) peut être un mélange d'acides carboxyliques C) différents (de structure différente).

En particulier, ledit composé amine A) peut être un mélange de composés amines A) tels que définis ci-haut et/ou ledit composé acrylate B) peut être un mélange de composés B) tels que définis ci-haut et/ou ledit composé acide C) peut être un mélange de composés C) tels que définis ci-haut. Egalement, ledit composé acide C) peut être un mélange, en particulier un mélange de monoacides éthyléniquement insaturés selon C11) ou un mélange de diacides éthyléniquement insaturés selon C21) ou un mélange de diacides saturés selon C22) ou un mélange desdits acides selon C11) et selon C21) et éventuellement selon C22).

Plus particulièrement, ledit composé amine A) peut être un mélange de composés amines A), tels que définis ci-haut et en particulier un mélange de composés amines selon A1) ou selon A2) ou un mélange de composés amines selon A1) et selon A2), avec lesdits composés selon A1) ou selon A2) étant tels que définis selon l'invention ci-dessus. De même, le composé acrylate B) peut être un mélange d'au moins un composé acrylate monofonctionnel, lequel en particulier peut être un mélange, de composés acrylates monofonctionnels et d'au moins un composé acrylate multifonctionnel, lequel en particulier peut être un mélange de composés acrylates multifonctionnels de nature chimique différente et de fonctionnalité identique ou différente ou de nature identique et de fonctionnalité différente.

Dans le cas de mélanges, la fonctionnalité à laquelle on se réfère correspond à la fonctionnalité moyenne en nombre $f_{moy}$ qui est calculée selon la formule (4) suivante :

$$f_{moy}= \Sigma_i \,(n_i{}^*f_i) \qquad (4)$$

avec $\Sigma_i\,(n_i)$ = 1 et $n_i$ étant la fraction en nombre de molécules i de fonctionnalité $f_i$.

Ledit oligomère précurseur P peut être défini en particulier selon la formule générale suivante (I) :

$$[[R_1\text{-}(R_2)N]_x\text{-}[R'_3\text{-}N(R'4)]_z\text{-}R_3\text{-}N(R_4)\text{-}CH_2CH_2\text{-}CO_2]_y\text{-}R_5\text{-}(CO_2\text{-}CH{=}CH_2)_{(n\text{-}y)} \qquad (I)$$

avec :

$R_1$, $R_2$ étant alkyles en $C_1$ à $C_4$ d'une amine tertiaire différente d'un aminoacrylate ou $R_1$-N= et $R_2$-N=, étant un groupement englobant une liaison aminoacrylate $R_6$-$O_2CCH_2CH_2$Net dans ce cas avec $R_1$ ou $R_2$ identiques ou différents et correspondant à $R_6$-$O_2CCH_2CH_2$, avec $R_6$ étant le résidu d'un composé monoacrylate, en particulier d'un monoacrylate de polyéther diol ou d'un monoacrylate de polyéther monoalcool.

$R_3$ : résidu dudit composé amine A), si porteur d'une fonction amine primaire et en option d'une fonction amine tertiaire, avec z = 0 et signifiant l'absence de fonction amine secondaire en présence de la fonction amine primaire,

$R'_3$ : est pour z = 0 et x = 1, une simple liaison entre $R_3$ et le groupement amine tertiaire « $R_1$-($R_2$)N- », ledit composé amine A) portant une fonction amine primaire et une tertiaire ou

$R'_3$ : est un alkylène en $C_2$ à $C_6$ pour z = 1 et x = 1 avec ledit composé amine A) portant une fonction amine primaire, une fonction amine secondaire et une fonction amine tertiaire, $R_4$ : pour z = 0, est un alkyle en $C_1$ à $C_4$ quand ledit composé amine A) porte une fonction amine secondaire et en option une fonction amine tertiaire,

sinon quand ledit composé amine A) porte une fonction amine primaire,

dans ce cas

$R_4$ : -$[CH_2CH_2\text{-}CO_2]_y$-$R_5$-($CO_2$-CH=$CH_2$)$_{(n-y)}$ ou

$R_4$ : au moins en partie est -$CH_2CH_2$-$CO_2$-$R'_5$ quand il y a présence d'acrylate monofonctionnel de radical $R'_5$ en plus de l'acrylate multifonctionnel, avec $R'_5$ pouvant comprendre un groupement hydroxy libre,

$R'_4$ : -$[CH_2CH_2\text{-}CO_2]_y$-$R_5$-($CO_2$-CH=$CH_2$)$_{(n-y)}$ quand z = 1 signifiant présence simultanée d'une fonction amine primaire avec une secondaire,

$R_5$ : résidu dudit composé acrylate B multifonctionnel sélectionné en particulier parmi les résidus de polyéthers, de polyesters ou parmi les résidus de polyuréthanes à base de polyesters et de polyéthers polyols,

x = 1 si l'amine A) porte une fonction amine tertiaire et 0 s'il y absence d'une telle fonction amine tertiaire,

z = 1 si présence de fonction amine secondaire en même temps que la fonction amine primaire dans le composé amine A) et z = 0 si absence de fonction amine secondaire,

n : fonctionnalité initiale dudit acrylate B) allant de 1 à 6, de préférence de 1 à 4,

y : nombre de groupements aminoacrylates créés par réactions d'addition d'un N-H dudit composé amine A) sur un groupement acrylate dudit acrylate B), avec 'y' allant de 1 à 6, de préférence de 1 à 4 et n-y : de 0 à 3 et de préférence de 0 à 2, représente le nombre de groupements acrylates résiduels dans ledit oligomère P.

Le choix de l'amine A) peut conditionner le choix de l'acrylate B). Selon une première option particulière, ledit composé amine A) est un composé amine selon A1) tel que défini selon l'invention ci-dessus et ledit composé acrylate B) comprend au moins un composé acrylate multifonctionnel selon B1) et au moins un composé acrylate monofonctionnel selon B2) et de préférence ledit composé acrylate multifonctionnel selon B1) est un ester multifonctionnel partiel ou complet d'au moins un polyéther polyol ou d'au moins un polyol dérivé dudit polyéther avec l'acide acrylique et ledit composé acrylate monofonctionnel selon B2) est un monoester de l'acide acrylique avec au moins un polyéther diol ou monool ou d'au moins un diol ou monool dérivé dudit polyéther, plus particulièrement lesdits polyéthers étant polyoxyéthylènes et ayant une masse moléculaire moyenne en nombre Mn inférieure à 2000 et de préférence inférieure à 1000. La Mn est calculée comme exposé ci-haut à partir de l'indice OH et la fonctionnalité desdits polyéthers polyols ou monools.

Selon une deuxième option particulière, ledit composé amine A) est un composé amine selon A2) tel que défini selon la revendication ci-dessus et de préférence ledit composé acrylate B) est un composé acrylate multifonctionnel selon B1) sélectionné parmi les esters partiels ou complets par l'acide acrylique d'un polyéther polyol ou d'un polyol dérivé dudit polyéther ou parmi les uréthanes acrylates à partir d'un polyéther polyol ou parmi les époxy acrylates à partir d'un glycidyl polyéther, plus particulièrement lesdits polyéthers étant des polyoxyéthylènes et ayant une masse moléculaire moyenne en nombre Mn inférieure à 2000 et de préférence inférieure à 1000. La Mn est calculée comme exposé ci-haut à partir de l'indice OH et la fonctionnalité desdits polyéthers polyols ou monools.

Plus particulièrement, la fonctionnalité desdits polyols comme base desdits acrylates multifonctionnels selon B1) peut varier de 2 à 6 et de préférence de 2 à 4. Le nombre d'unités éthoxy par fonction hydroxy dudit polyol pour les polyols éthoxylés peut varier de 2 à 20 et de préférence de 3 à 20 et plus préférentiellement de 5 à 20.

Les uréthanes acrylates selon B1) à partir de polyéther polyols peuvent être obtenus par réaction d'un polyéther polyol, en particulier d'un polyoxyéthyléne polyol avec un précondensat monoisocyanate obtenu par réaction d'un hydroxyalkyl acrylate tel que l'hydroxy éthyl acrylate (HEA) avec un diisocyanate.

Les époxy acrylates selon B1), à partir de glycidyle polyéthers multifonctionnels (éthers de glycidyle d'un polyéther polyol), en particulier éthers de glycidyle de polyoxyéthyléne polyols, peuvent être obtenus par réaction desdits éthers de glycidyle de polyéther polyols (polyéthers époxydés) avec l'acide acrylique.

Comme exemples convenables de polyols éthoxylés pour des esters acrylates multifonctionnels selon B1), on peut citer le triméthylol propane éthoxylé (3 OH), le pentaérythritol éthoxylé (4 OH), le di triméthylol propane éthoxylé (4 OH), le dipentaérythritol éthoxylé (6 OH), sorbitol éthoxylé (3 OH), oligoesters polyols éthoxylés, polyoxyéthylène polyols, le bisphénol A éthoxylé (2 OH), l'isosorbide éthoxylé (2 OH), tricyclodecanedimethanol éthoxylé (2 OH), néopenthylglycol éthoxylé (2 OH), glycérol éthoxylé (3 OH). Ces polyols éthoxylés conviennent parfaitement pour obtenir par acrylation complète ou partielle (suivant le cas si fonctionnalité OH d'au moins 3) les esters acrylates multifonctionnels selon B1). Plus particulièrement, l'acrylate multifonctionnel selon B1) peut être un polyéthylèneglycol diacrylate de Mn variable allant de 150 à 600, le bisphénol A éthoxylé diacrylate avec un nombre d'unité éthoxy allant de 4 à 15 tels que SR 349, SR 601, SR 602 de Sartomer ou le triméthylolpropane éthoxylé triacrylate avec un nombre d'unités éthoxy allant de 3 à 20 tels que SR 454, SR 499, SR 502, SR 9035 ou SR 415 de Sartomer ou le glycérol éthoxylé triacrylate avec 3 à 15 éthoxy tel que SR 9046 de Sartomer ou le pentaérythritol éthoxylé tetraacrylate avec 3 à 15 éthoxy tel que le SR 494 de Sartomer.

Comme composé amine A), on peut distinguer pour le composé amine selon A1), tel que défini ci-haut, les catégories suivantes :

A11) Monoamines primaires, avec comme exemples de monoamines primaires pouvant convenir, les amines suivantes : les N-alkylamines telles que l'amylamine, 2-aminopentane, 3-aminopentane, 1,2-diméthylpropylamine, hexylamine, 1,3-diméthylbutylamine, n-heptylamine, n-octylamine, 2-aminooctane, 3,3,5-trimethylcyclohexylamine, éthylamine, isopropylamine, sec-butylamine, les N-alkylamines porteuses de groupements hydroxyles telles que Alaninol, les N-alkoxyamines telles que 3-éthoxypropylamine, 3-(2-méthoxyéthoxy) propylamine, 3-butoxypropylamine, 3-(2-éthylhexyloxy) propylamine, 3-Isopropoxypropylamine (IPOPA), 3-méthoxypropylamine (MOPA), polyéther amines telles que Jeffamine® M600, M1000 commercialisées par Hunstman, les amines porteuses de fonctions spécifiques telles que la 1-(2-aminoéthyl)-2-imidazolidinone appelée aussi UDETA (urée diéthylène triamine en anglais « urea diethylene triamine »), composés amides-aminés (à partir d'un diacide et d'une diamine primaire en excès) ou composés esters-amides aminés (à partir d'un ester acide avec une diamine primaire en excès),

A12) Les diamines primaire-secondaires avec comme exemples convenables les N-alkylaminoalkylamines telles que la N-méthyl-1,3-diaminopropane, N-propyl-1,3-propane diamine, N-isopropyl-1,3-propane diamine, N-cyclohexyl-1,3-propanediamine, triacétone diamine, 1,2-diaminocyclohexane (1,2-DACH), bis(4-aminocyclohexyl)méthane (PACM) ou les N-alkylaminoalkylamines porteuses de groupements hydroxyles, en particulier les hydroxyalkyl aminoalkylamines, telles que 2-(3-aminopropylamino) éthanol,

A13) Les diamines primaire-primaires avec comme exemples convenables qu'on peut citer: les alkylènediamines ou cycloalkylènediamines telles que la 1,3-Bis(aminométhyl)cyclohexane (1,3-BAC), isophorone diamine (IPDA) ou les polyéther diamines avec 2 à 20 unités éthers, en particulier éthoxy et/ou propoxy telles que les Jeffamine® commercialisées par Hunstman sous les noms commerciaux D230, D400, ED600, ED900, EDR148, EDR 176, EDR 104, THF 100, THF 140, THF 230, RFD 270 ou les amines grasses dérivées des dimères d'acides gras en $C_{36}$ telles que commercialisées par Croda sous le nom commercial les Priamines®,

A14) Les diamines primaires-tertiaires avec comme exemples convenables qu'on peut citer : les N,N dialkylaminoalkylamines telles que la diéthylaminopropylamine (DEAPA), diméthylaminopropylamine (DMAPA), 3-(dibutylamino)propylamine, N,N-diméthyl éthylène diamine ou 1-(3-aminopropyl)-2-pyrrolidine, 3-morpholinopropylamine, 1-(3-aminopropyl)pipéridine, 1-(3-aminopropyl)-2-pipécoline, les N,N dialkylaminoalkylamines porteuses de groupements hydroxyles, en particulier dihydroxyalkyl aminoalkyl amines telles que la N-(aminopropyl)diéthanolamine (APDEA),

A15) Les triamines primaire-secondaire-tertiaires avec comme exemples convenables qu'on peut citer: la N,N-diméthyldipropylène triamine (DMAPAPA), N-aminoéthylpipérazine, 3-(2-aminoéthyl)aminopropylamine, Bis(3-aminopropyl)amine, méthyl bis(3-aminopropyl)amine, N-(3-Aminopropyl)-1,4-diaminobutane.

Comme composé amine A), on peut distinguer pour le composé amine selon A2), tel que défini ci-haut, les catégories suivantes :

A21) les monoamines secondaires avec comme exemples convenables les N,N dialkylamines telles que la di-sec-butylamine, la diamylamine, l'isopropylbenzylamine, la dihexylamine, la diéthylamine, la diisopropylamine, N-isopropylméthylamine, la N butylméthylamine, N-sec-butylméthylamine, N-isobutylméthylamine, N-ter-butylméthylamine, N-méthylpentylamine, N-hexylméthylamine, N-méthylcyclohexylamine, N-heptylméthylamine, N-octylméthylamine, N-éthylméthylamine, N-éthylpropylamine, N-éthylisopropylamine, N-butyléthylamine, N-sec-butyléthylamine, N-éthylcyclohexylamine, N-éthylbenzylamine, les N,N dialkylamines porteuses de groupements hydroxyles telles que 2-(éthylamino)éthanol, 2-(isopropylamino)éthanol, 2-butylaminoéthanol, 2-benzylaminoéthanol, 2-octylaminoéthanol, 2-sec-butylaminoéthanol,

A22) Les diamines secondaire-secondaire avec comme exemples convenables à citer : les N, N' dialkyl alkyl diamines telles que la N, N' diméthyl éthylène diamine ou les N, N' dialkyl cycloalkylène diamines ou bis(N, N' dialkyl cycloalkylène) alkylène telles que les diamines secondaires cycloaliphatiques sous le nom commercial JEFFLINK® 136 et 754 commercialisées par Hunstman,

A23) Les diamines secondaire-tertiaire avec comme exemples convenables à citer les N,N,N'-triméthyl-1,3-propanediamine ou les adduits aminés époxy-amine.

Sont préférés comme composé amine A), les composés amines selon A1) et plus particulièrement les composés amines : les diamines selon A12) primaire-secondaire, selon A13) primaire-primaire, selon A14) primaire-tertiaire ou les triamines selon A15) primaire-secondaire-tertiaire et encore plus particulièrement les diamines selon A13) ou selon A14) et les triamines selon A15). Selon une option plus particulière, ledit composé amine A) est selon A1) et choisi selon A14).

Concernant la fonctionnalité dudit oligomère, de préférence il porte comme insaturation éthylénique, des groupements acrylates avec une fonctionnalité en groupements acrylates, y compris acrylate d'ammonium si l'acide acrylique est utilisé comme composé acide carboxylique C), allant de 1 à 6, de préférence de 1 à 4, plus préférentiellement de 1 à 3. Plus particulièrement, ledit oligomère selon l'invention porte au moins 2 groupements acrylates par oligomère et est donc plus particulièrement réticulable, ce qui veut dire seul sans besoin d'agent réticulant.

Concernant le taux de sites d'amines tertiaires, y compris aminoacrylates formés par addition dudit composé amine A) sur ledit acrylate B), qui sont converties en sel de carboxylate d'ammonium, il peut être représenté par le taux de fonctions amines tertiaires ainsi salifiées. De préférence, l'oligomère selon l'invention à un taux de fonctions amines tertiaires salifiées allant de 0,1 à 25, de préférence de 0,5 à 15 mEq par g dudit oligomère. De préférence, ledit oligomère a une masse moléculaire moyenne en nombre Mn calculée par le bilan matière, inférieure à 5000, de préférence inférieure à 3000.

L'invention couvre également l'oligomère précurseur P, précurseur de l'oligomère tel que défini selon l'invention ci-avant, en particulier comprenant ou étant un oligomère selon la formule (I) telle que définie ci-avant.

Un deuxième objet de l'invention concerne une solution d'oligomère dans un diluant réactif, laquelle comprend l'oligomère tel que défini ci-haut selon l'invention et au moins un diluant réactif D) sélectionné parmi les mono (méth)acrylates et/ou les (méth)acrylates multifonctionnels, de préférence avec D) étant un (méth)acrylate d'un polyéther monool ou polyol ou d'un dérivé de polyéther monool ou polyol, de masse moléculaire moyenne en nombre Mn inférieure ou égale à 600. Cette masse Mn est calculée à partir de $I_{OH}$ et la fonctionnalité dudit monool ou polyol et sa fonctionnalité comme exposé ci-haut.

Un autre objet de la présente invention concerne un procédé de préparation dudit oligomère tel que défini ci-haut selon l'invention, lequel comprend les étapes successives suivantes :

i) Préparation dudit oligomère précurseur P par l'addition d'au moins un composé amine A), selon A1) et/ou selon A2) tel que défini ci-haut pour A) selon l'invention, sur
B) un composé acrylate parmi :

- B1) au moins un acrylate multifonctionnel ayant une fonctionnalité, en particulier fonctionnalité moyenne en nombre si mélange, allant de 2 à 6 et de préférence de 2 à 4, plus préférentiellement de 2 à 3, encore plus préférentiellement de 2 quand ledit composé amine A) est un composé selon A2) tel que défini ci-haut pour le composé amine A) ou
- un mélange d'au moins un acrylate multifonctionnel selon B1) comme défini ci-dessus et d'au moins un acrylate monofonctionnel selon B2), quand ledit composé amine A) est un composé amine selon A1), tel que défini ci-haut pour le composé amine A), ceci pour bloquer par l'aminoacrylate formé un des deux groupements -NH de ladite fonction amine primaire dudit composé amine selon A1),

avec les rapports molaires suivants entre groupements :
(acrylate) / (-NH) > 1 de sorte qu'au moins un groupement acrylate résiduel est porté par le produit d'addition i) correspondant audit oligomère précurseur P et dans le cas où ledit composé amine A) est selon A1), ledit acrylate B) est un mélange d'acrylate multifonctionnel selon B1) et d'acrylate monofonctionnel selon B2) avec un rapport molaire B2 / A1 = 1/1
ii) Salification dudit oligomère précurseur P qui est le produit de l'étape d'addition i), sous forme de sel de carboxylate d'ammonium par ledit composé acide carboxylique C), tel que défini ci-haut selon l'invention, de préférence avec un rapport molaire entre groupements carboxy et fonctions amine tertiaire (=N-) y compris amines tertiaires parmi groupements aminoacrylates, (carboxy)/(amine tertiaire) allant de 0,10 à 1,00, de préférence de 0,5 à 1,00 et plus préférentiellement de 0,5 à 0,99.

Fait également partie de l'invention, un oligomère polymérisable, en particulier réticulable, tel qu'obtenu par le procédé tel que défini ci-dessus selon l'invention.

Un autre objet de l'invention selon les revendications 21 et 22 concerne une composition polymérisable, en particulier réticulable, laquelle comprend comme liant au moins un oligomère tel que défini ci-dessus selon l'invention ou obtenu par un procédé tel que défini ci-haut selon l'invention ou une solution telle que définie ci-dessus selon la présente invention.

Plus particulièrement, ladite composition est polymérisable, en particulier réticulable, par :

- voie de rayonnement, en particulier UV, LED, laser, faisceau d'électrons, de préférence UV,
- voie thermique ou de peroxyde ou d'hydroperoxyde en présence d'un accélérateur,

- voie duale combinant au moins deux des voies précitées,
  et, de préférence,
- par voie de rayonnement, plus préférentiellement par voie UV.

Encore plus particulièrement, il s'agit d'une composition polymérisable, en particulier réticulable, pour matériau à fonction provisoire, de préférence parmi revêtement, en particulier encre, vernis, adhésif ou parmi hydrogel ou matériau support pour objet d'impression en 3D couche par couche, plus préférentiellement revêtement ou matériau support pour objet d'impression en 3D couche par couche et encore plus préférentiellement pour matériau de support d'objet d'impression en 3D couche par couche.

Un autre objet de la présente invention concerne l'utilisation d'un oligomère tel que défini ci-haut selon l'invention ou d'une solution d'oligomère telle que définie selon l'invention ou l'utilisation de l'oligomère obtenu par un procédé tel que défini selon l'invention, en tant que liant polymérisable et en particulier réticulable, en option hydrosoluble, dans des compositions polymérisables et en particulier réticulables, de préférence sous rayonnement. Ladite utilisation s'applique en particulier à un matériau à fonction provisoire, éliminable par lavage à l'eau seule ou par une eau saline ou par une autre solution aqueuse, de préférence ayant un pH > 7, plus préférentiellement > 8, en particulier à des matériaux à fonction provisoire parmi les revêtements, hydrogels ou un matériau support pour objet d'impression en 3D couche par couche, plus particulièrement revêtements ou matériau support pour objet d'impression en 3D couche par couche.

Encore plus particulièrement, ladite utilisation s'applique spécifiquement à l'impression des objets 3D par polymérisation, en particulier réticulation, sous rayonnement couche par couche, d'une composition comprenant ledit oligomère ou ladite solution d'oligomère comme matériau à fonction provisoire de support ou de consolidation ou de moulage dudit objet 3D final, obtenu après élimination dudit matériau à fonction provisoire par lavage à l'eau ou par une solution saline ou par une autre solution aqueuse.

Une autre possibilité d'utilisation concerne des hydrogels obtenus à partir de la réticulation d'une composition réticulable comprenant ledit oligomère ou ladite solution d'oligomère suivie d'un gonflement avec l'eau ou une solution aqueuse convenable. Une telle utilisation peut en particulier concerner le transport de principes actifs pharmaceutiques ou phytosanitaires ou pour le traitement de bois en utilisant lesdits hydrogels comme véhicule ou vecteur dudit principe actif pharmaceutique, phytosanitaire ou pour le traitement de bois.

Finalement, fait également partie de la présente invention, un matériau polymère, en particulier réticulé, lequel résulte de l'utilisation comme liant polymérisable, en particulier réticulable d'au moins un oligomère selon l'invention tel que défini ci-haut ou d'un oligomère obtenu par le procédé de l'invention tel que défini ci-avant ou d'une solution d'oligomère selon l'invention telle que définie ci-dessus ou de la polymérisation, en particulier de la réticulation, d'une composition polymérisable, en particulier réticulable selon l'invention, telle que définie ci-haut.

Plus particulièrement, ledit matériau est un matériau à fonction provisoire, éliminable par l'eau, une solution saline ou une autre solution aqueuse adaptée à cette élimination, par exemple par ajustement du pH à > 7, en particulier pH à > 8, de préférence parmi les revêtements, hydrogels ou un matériau de support ou de consolidation ou de moulage d'un objet imprimé en 3D couche par couche sous rayonnement et plus préférentiellement un matériau provisoire de support ou de consolidation ou de moulage d'un objet imprimé en 3D couche par couche sous rayonnement.

Les exemples suivants sont présentés pour illustrer l'invention et ses performances et ne limitent en rien sa portée.

EXEMPLES

**1) Préparation et formulation d'oligomères selon l'invention**

Exemple 1

Dans un réacteur de 1 L, on introduit 108,54 g de diméthylaminopropylamine (DMAPA de Huntstman, Mw de 102,18 g/mol) et 0,14 g de 2,6-di-tert-butyl-4-méthylphénol (BHT). Sous agitation et bullage d'air, 356,93 g de polyéthylène glycol monoacrylate (Bisomer PEA6 de Geo Specialty Chemicals, Mw de 336 g/mol) sont ajoutés à température ambiante sur une heure à débit constant. Une exothermie d'environ 20°C est observée. A la fin de l'ajout, la température du mélange est portée à 60°C. Après quatre heures à 60°C, 464,22 g de Butane diol diglycidyl éther diacrylate (BDDGEDA, CN132 de Sartomer, Mw de 256,8 g/mol) sont ajoutés au mélange en trente minutes à débit constant. A la fin de l'ajout, la température du mélange est maintenue à 60°C pendant trois heures, puis 70,17 g d'acide glutarique (Aldrich, 132,11 g/mol) sont ajoutés au mélange. A la fin de l'ajout, on observe un temps supplémentaire de réaction d'une heure à 60°C avant de récupérer le produit final.

Exemple 2

Dans un réacteur de 1 L, on introduit 125,68 g de diméthylaminopropylamine (DMAPA de Huntstman, Mw de 102,18 g/mol) et 0,93 g de 2,6-di-tert-butyl-4-méthylphénol (BHT). Sous agitation et bullage d'air, 413,27 g de polyéthylène glycol monoacrylate (Bisomer PEA6 de Geo Specialty Chemicals, Mw de 336 g/mol) sont ajoutés à température ambiante sur une heure à débit constant. Une exothermie d'environ 20°C est observée. A la fin de l'ajout, la température du mélange est portée à 60°C. Après quatre heures à 60°C, 371,45 g de polyéthylène glycol diacrylate (SR259 de Sartomer, Mw de 302 g/mol) sont ajoutés au mélange en trente minutes à débit constant. A la fin de l'ajout, la température du mélange est maintenue à 60°C pendant trois heures puis 88,68 g d'acide acrylique (Arkema, Mw de 72,1 g/mol) est ajouté.

A la fin de l'ajout, on observe un temps supplémentaire de réaction d'une heure à 60°C avant de récupérer le produit final.

Exemple 3

Dans un réacteur de 1 L, on introduit 131,16 g de diméthylaminopropylamine (DMAPA de Huntstman, Mw de 102,18 g/mol) et 1,01 g de 2,6-di-tert-butyl-4-méthylphénol (BHT). Sous agitation et bullage d'air, 775,29 g de polyéthylène glycol diacrylate (SR 259 de Sartomer, Mw de 302 g/mol) sont ajoutés à température ambiante sur une heure à débit constant. Une exothermie d'environ 20°C est observée. A la fin de l'ajout, la température du mélange est portée à 60°C. Après quatre heures à 60°C, 92,55 g d'acide acrylique (Arkema, Mw de 72,1 g/mol) sont ajoutés sur trente minutes à débit constant. A la fin de l'ajout, on observe un temps de réaction d'une heure à 60°C avant de récupérer le produit final.

Exemple 4

Dans un réacteur de 1 L, on introduit 80,89 g de diméthylaminopropylamine (DMAPA de Huntstman, Mw de 102,18 g/mol) et 0,831 g de 2,6-di-tert-butyl-4-méthylphénol (BHT). Sous agitation et bullage d'air, 554,13 g de polyéthylène glycol monoacrylate (Bisomer PEA6 de Geo Specialty Chemicals, Mw de 336 g/mol) sont ajoutés à température ambiante sur une heure à débit constant. Une exothermie d'environ 20°C est observée. A la fin de l'ajout, la température du mélange est portée à 60°C. Après quatre heures à 60°C, 114,15 g d'acide acrylique (Arkema, Mw de 72,1 g/mol) sont ajoutés au mélange en trente minutes à débit constant. A la fin de l'ajout, on observe un temps de réaction d'une heure à 60°C. 250 g de polyéthylène glycol diacrylate (SR 344 de Sartomer) comme monomère de dilution ou diluant réactif, sont alors ajoutés. Un temps supplémentaire d'homogénéisation de trente minutes à 60°C est observé avant de récupérer le produit final.

**2) Exemples comparatifs**

Comme référence de comparaison, on utilise des compositions réticulables représentatives de l'état de la technique comprenant, en plus des composants réactifs sans aminoacrylates, des composants non réactifs hydrosolubles tels que le polyéthylène glycol 600 (PEG 600).

Exemple 5

Dans un réacteur de 1 L, on introduit 626 g de polyéthylène glycol de masse 600, 368 g de polyéthylène glycol de masse 600 diacrylate (SR 610 de Sartomer) et 6 g de glycérol propoxylée triacrylate (SR 9020 de Sartomer). Sous agitation et bullage d'air, le mélange est porté à 60°C. Un temps de mélange de trente minutes à 60°C est observé.

Exemple 6

Dans un réacteur de 1 L, on introduit 337 g de polyéthylène glycol de masse 600, 342 g de polyéthylène glycol de masse 600 diacrylate (SR 610 de Sartomer), 315 g de polyéthylène glycol monoacrylate (Bisomer PEA6 de Geo Specialty Chemicals, Mw de 336 g/mol) et 6 g de glycérol propoxylée triacrylate. Sous agitation et bullage d'air, le mélange est porté à 60°C. Un temps de mélange de trente minutes à 60°C est observé.

**3) Caractéristiques des compositions avant réticulation**

Tableau 1

| Caractéristiques | Unité | Compositions selon invention (produits tels que décrits dans exemples 1 à 4) | | | | Compositions comparatives | |
|---|---|---|---|---|---|---|---|
| | | 1 | 2 | 3 | 4 | 5 | 6 |
| Viscosité Brookfield à 25°C | mPa.s | 40000 | 2091 | 1636 | 11360 | 150 - 200 | 150 - 200 |
| Viscosité Brookfield à 90°C | mPa.s | 1020 | 52 | 43 | 38 | 10 - 15 | 10-15 |

**4) Evaluation des propriétés des compositions réticulés et des performances d'hydro-fragmentation dans une solution aqueuse**

Pour les tests de compression et d'hydro-fragmentation, les produits tels que décrits selon l'invention (Exemples 3 et 4) et les compositions comparatives (Exemples 5 et 6) sont formulés avec un taux global de photoinitiateur de 5% en poids composé de 4% d'Irgacur® 1173 (BASF) et de 1% en poids de Lucirin® TPO-L (BASF) pour 95% en poids de composition testée du Tableau 1. La réticulation est réalisée sous lampe UV 120 Watt/cm.

Test de compression (suivant la norme NF EN ISO 604)

Type d'éprouvettes : Cylindrique (diamètre : 13,3 mm, hauteur 26,5 mm) obtenues par réticulation sous lampe UV 120 Watt/cm d'une composition contenue dans un moule cylindrique en verre sacrifié ayant les dimensions (diamètre) correspondant à celles de l'éprouvette (longueur ajustée par découpage de l'objet réticulé).
Machine d'essai : INSTRON 1185 ReNew, cellule 10kN
Vitesse de l'essai : 1,3 mm/min.
Les éprouvettes ont été testées à 23°C.
Les résultats sont présentés dans le Tableau 2 ci-dessous.

Tableau 2

| Caractéristique | Unité | Composition selon invention | | Composition comparative | |
|---|---|---|---|---|---|
| | | Exemple 3 | Exemple 4 | Exemple 5 | Exemple 6 |
| Module Young | MPa | 7,437 | 3,548 | 0,695 | 2,489 |

Test d'hydro-fragmentation

Le test d'hydro-fragmentation est défini de la façon suivante :
Un objet réticulé en masse sous la forme d'un cylindre de 15 g et de 15 cm$^3$ (2 cm de haut, 1,5 cm de rayon) est plongé pendant deux heures dans un bain contenant 400 ml d'une solution aqueuse d'hydrogénocarbonate de sodium (pH > 7) à 60°C, sous agitation magnétique (barreau magnétique). Après deux heures de traitement, le mélange est filtré et les solides résiduels séchés.

Deux critères sont alors mesurés et évalués :

1) Le poids du cylindre avant le test, puis après filtration et séchage, indique la partie de l'objet passée en solution, « hydrosoluble » ou « hydrodispersible »
2) L'aspect du cylindre après le test : une note de 0 à 5 est attribuée ; elle indique le degré « hydro-fragmentabilité » du matériau.

a. 0 : le cylindre original est resté intact
b. 5 : le cylindre est entièrement fragmenté, les résiduels solides se présentant sous forme de poudre homogène après filtration et séchage.

Les objets cylindriques préparés pour le test sont obtenus à partir d'un moule en Téflon. 20 passages sous une lampe UV de 120 Watt/cm sont nécessaires pour obtenir l'objet réticulé. Les résultats sont présentés dans le Tableau 3 ci-dessous.

Tableau 3

| | Unité | Composition selon invention | | Composition comparative | |
|---|---|---|---|---|---|
| | | Exemple 3 | Exemple 4 | Exemple 5 | Exemple 6 |
| Masse objet avant test | 9 | 15,6 | 15,0 | 14,8 | 14,7 |
| Fraction solide «résiduelle» filtrée sur papier filtre | 9 | 7,8* | 5,25** | 14,69*** | 12,5*** |
| Fraction passant dans la phase aqueuse et à travers le filtre papier standard | % | 50 | 65 | 1 | 15 |
| Degré d'«hydro-fragmentabilité » | | 4 - 5 | 5 | 0 | 2 |
| * : particules de taille homogène avec taille maximale inférieure à 7 mm<br>** : particules de taille homogène avec taille maximale inférieure à 5 mm<br>*** : particules de taille très variable (très hétérogènes) de taille maximale majoritaire et supérieure à 20 mm | | | | | |

## Exemples supplémentaires d'oligomères

### Exemple 7

Dans un réacteur de 1 L, on introduit 808,66 g de méthoxy polyéthylène glycol monoacrylate (SR 551 de Sartomer, Mw de 404 g/mol), 0,983 g de 2,6-di-tert-butyl-4-méthylphénol (BHT) et 0,983 g d'éther méthylique d'hydroquinone (EMHQ). Sous agitation et bullage d'air, sont ajoutés à température ambiante sur trente minutes à débit constant 78,80 g de diméthyl aminopropylamine (DMAPA de Huntstman, Mw de 102,18 g/mol). Une exothermie d'environ 10°C est observée. A la fin de l'ajout, la température du mélange est portée à 50°C. Après quatre heures à 50°C, 110,08 g d'acide acrylique (Arkema, Mw de 72,1 g/mol) sont ajoutés au mélange à débit constant sur trente minutes. La réaction est fortement exothermique, le débit de l'ajout est ajusté afin de ne pas dépasser la température de 55°C dans le milieu. A la fin de l'ajout, on observe un temps de réaction de deux heures à 50°C. Après deux heures à 50°C, 0,494 g de phénothiazine sont ajoutés au mélange.

### Exemple 8

Dans un réacteur de 1 L, on introduit 791,77 g de méthoxy polyéthylène glycol monoacrylate (SR 551 de Sartomer, Mw de 404 g/mol), 0,964 g de 2,6-di-tert-butyl-4-méthylphénol (BHT) et 0,964 g d'éther méthylique d'hydroquinone (EMHQ). Sous agitation et bullage d'air, sont ajoutés à température ambiante sur trente minutes à débit constant 98,19 g de diméthyl aminopropylamine (DMAPA de Huntstman, Mw de 102,18 g/mol). Une exothermie d'environ 10°C est observée. A la fin de l'ajout, la température du mélange est portée à 50°C. Après quatre heures à 50°C, 107,63 g d'acide acrylique (Arkema, Mw de 72,1 g/mol) sont ajoutés au mélange à débit constant sur trente minutes. La réaction est fortement exothermique, le débit de l'ajout est ajusté afin de ne pas dépasser la température de 55°C dans le milieu. A la fin de l'ajout, on observe un temps de réaction de deux heures à 50°C. Après deux heures à 50°C, 0,482 g de phénothiazine sont ajoutés au mélange.

### Exemple 9

Dans un réacteur de 1 L, on introduit 388,40 g de tertiobutyl cyclohexanol acrylate (SR 217 de Sartomer, Mw de 210/mol), 0,98 g d'éther méthylique d'hydroquinone (EMHQ) et 0,98 g de 2,6-di-tert-butyl-4-méthylphénol (BHT). Sous agitation et bullage d'air, 147,67 g de diméthylaminopropylaminopropylamine (DMAPAPA d'Aldrich, Mw de 159,27 g/mol) sont ajoutés à température ambiante sur une heure à débit constant. Une exothermie d'environ 10°C est observée. A la fin de l'ajout, la température du mélange est portée à 50°C. Après quatre heures à 50°C, 280 g de polyéthylène glycol diacrylate (SR 259 de Sartomer, Mw de 302 g/mol) sont ajoutés sur une heure à débit constant. Après deux heures à 50°C, 180,48 g d'acide acrylique (Arkema, Mw de 72,1 g/mol) sont ajoutés au mélange en trente minutes à débit constant. Le débit d'introduction est ajusté afin de ne pas dépasser une température de 55°C dans le milieu. A la fin de l'ajout, on observe un temps de réaction deux heures à 50°C. Après deux heures à 50°C, 0,49 g de phénothiazine sont ajoutés au mélange.

Tableau 4 : Caractéristiques des produits des exemples 7 à 9

| Caractéristiques | Unité | Exemple 7 | Exemple 8 | Exemple 9 |
|---|---|---|---|---|
| Viscosité Brookfield à 25°C | mPa.s | 353 | 147 | 35600 |
| Viscosité Brookfield à 85°C | mPa.s | 19,0 | 15,8 | 836 |
| Module de Young | MPa.s | 1,0* | 0,7* | 3,5 |
| *Mesures réalisées avec: 20% de SR 344 (Polyéthylène Glycol 400 Diacrylate de Sartomer)* | | | | |

**Revendications**

1. Oligomère polymérisable, **caractérisé en ce qu'**il est porteur d'au moins une insaturation éthylénique et d'au moins une liaison ionique de carboxylate d'ammonium et **en ce qu'**il comprend dans sa structure ou dans sa composition :

   P) au moins un oligomère précurseur, porteur d'au moins une fonction amine tertiaire sous forme de groupement aminoacrylate, résultant de l'addition de A) au moins un composé amine selon A1) porteur d'au moins une fonction amine primaire ($-NH_2$) et/ou secondaire ($-NH$) et en option d'au moins une fonction amine tertiaire et/ou selon A2) porteur d'au moins une fonction amine secondaire ($-NH$) et en option d'au moins une fonction amine tertiaire sur B) au moins un composé acrylate hydrophile avec formation ainsi dudit au moins un groupement aminoacrylate et
   C) au moins un composé acide carboxylique attaché audit oligomère précurseur P par au moins une liaison ionique de carboxylate d'ammonium avec au moins un desdits groupements aminoacrylates et en option au moins une desdites fonctions amines tertiaires dudit composé amine A).

2. Oligomère selon la revendication 1, **caractérisé en ce que** ledit composé amine A) est sélectionné parmi :

   A1) un composé amine, porteur d'au moins une fonction amine primaire et en option, d'au moins une fonction amine tertiaire et/ou d'au moins une fonction amine secondaire et/ou
   A2) un composé amine porteur d'au moins une fonction amine secondaire et en option d'au moins une fonction amine tertiaire,
   et **en ce que** ledit composé acrylate B) est au moins un composé acrylate multifonctionnel selon B1) et/ou monofonctionnel selon B2) et en particulier pour A) étant ou comprenant un composé amine selon A1), ledit composé acrylate B) est un mélange d'au moins un acrylate multifonctionnel selon B1) et d'au moins un acrylate monofonctionnel selon B2).

3. Oligomère selon la revendication 2, **caractérisé en ce que** ledit composé acrylate hydrophile B) est un mélange de composé acrylate multifonctionnel selon B1) et de composé acrylate monofonctionnel selon B2).

4. Oligomère selon l'une des revendications 1 à 3, **caractérisé en ce que** ledit groupement aminoacrylate peut porter au moins une insaturation éthylénique ou aucune insaturation éthylénique et, dans ce dernier cas, ledit composé acide C) est éthyléniquement insaturé.

5. Oligomère selon l'une des revendications 1 à 4, **caractérisé en ce que** ledit composé acide C) est un monoacide selon C1) qui est éthyléniquement insaturé selon C11) et ledit oligomère porte au moins deux liaisons ioniques carboxylates d'ammonium.

6. Oligomère selon l'une des revendications 1 à 5, **caractérisé en ce que** :

   - quand ledit composé acide carboxylique C) est selon C1) un monoacide, dans ce cas :

     C1) est un monoacide selon C11) éthyléniquement insaturé et au moins une insaturation éthylénique dudit oligomère est portée par au moins ledit monoacide selon C11) par liaison carboxylate d'ammonium avec au moins une desdites fonctions amines tertiaires parmi lesdits groupements aminoacrylates ou en option parmi les fonctions amines tertiaires portées dudit composé amine A) et éventuellement au moins une autre insaturation éthylénique dudit oligomère est portée par au moins un groupement aminoacrylate sous forme de groupement aminoacrylate-acrylate terminal dudit oligomère précurseur P ou
C1) est un monoacide selon C12) saturé et au moins une insaturation éthylénique dudit oligomère est portée par au moins un groupement aminoacrylate sous forme de groupement aminoacrylate-acrylate terminal dudit oligomère précurseur P et

- quand ledit composé acide carboxylique C) est selon C2) un polyacide carboxylique et dans ce cas :

C2) est un polyacide selon C21) éthyléniquement insaturé, et au moins une insaturation éthylénique dudit oligomère est portée par au moins ledit polyacide selon C21) et éventuellement en plus, au moins une insaturation éthylénique est portée par au moins un groupement aminoacrylate sous forme de groupements aminoacrylate-acrylate terminal dudit oligomère précurseur P ou
C2) est un polyacide selon C22) saturé et ledit oligomère précurseur P porte au moins un groupement aminoacrylate-acrylate terminal et ledit oligomère qui résulte porte au moins deux insaturations éthyléniques acrylates.

7. Oligomère selon la revendication 6, **caractérisé en ce que** ledit composé acide carboxylique C) est un diacide insaturé selon C21) ou saturé selon C22) associant par deux liaisons ioniques carboxylates, deux molécules dudit oligomère précurseur P, par salification sous forme de sel de carboxylate d'ammonium d'une desdites fonctions amines tertiaires parmi lesdits groupements aminoacrylates formés ou en option parmi lesdites fonctions amines tertiaires dudit composé amine A), sur chacune desdites molécules dudit oligomère P ou **en ce que** ledit composé acide carboxylique C) est un monoacide selon C1), éthyléniquement insaturé selon C11) ayant une insaturation éthylénique polymérisable et **en ce que** ledit monoacide selon C11) salifie au moins deux desdites fonctions amines tertiaires sous forme de carboxylate d'ammonium.

8. Oligomère selon l'une des revendications 1 à 7, **caractérisé en ce que** ledit oligomère précurseur P comprend dans sa structure, des segments de chaîne hydrophiles, sélectionnés parmi les polyéthers ou parmi les polyesters, ou parmi les polyuréthanes à base d'oligoéthers polyols ou de polyols éthoxylés, lesdits segments ayant une masse moléculaire moyenne en nombre Mn inférieure à 2000.

9. Oligomère selon l'une des revendications 1 à 8, **caractérisé en ce que** ledit composé acide carboxylique C) est selon C11) un monoacide éthyléniquement insaturé et sélectionné parmi : acide acrylique ou méthacrylique, acide crotonique (acide trans-2-buténoïque) ou le $\beta$-carboxyéthyl acrylate ($\beta$-CEA) ou leurs mélanges.

10. Oligomère selon l'une des revendications 1 à 8, **caractérisé en ce que** ledit composé acide carboxylique C) est selon C22) un diacide carboxylique saturé et sélectionné parmi : acide succinique, acide malonique, acide malique, acide glutarique (en $C_5$ : porteur de 5 atomes de carbone), acide adipique (en $C_6$), acide pimélique (en $C_7$) ou diester-acides de diacides précités avec un alcane diol en $C_2$ à $C_4$ ou avec un di-, tri- ou tetra-éthylène glycol ou diacides parmi les dimères et/ou trimères d'acides gras, ou leurs mélanges par deux ou par trois.

11. Oligomère selon l'une des revendications 1 à 8, **caractérisé en ce que** ledit composé acide carboxylique C) est selon C21) un diacide carboxylique insaturé et sélectionné parmi : acide itaconique, acide maléique, acide fumarique, acide tetrahydrophtalique (acide cyclohexène dioïque) ou diesters acides des diacides précités avec un alcane diol en $C_2$ à $C_4$ ou avec un di-, tri- ou tetra-éthylène glycol ou les dimères et/ou trimères d'acides gras non hydrogénés avec dimères en $C_{36}$ et trimères en $C_{54}$ ou leurs mélanges.

12. Oligomère selon l'une des revendications 1 à 8, **caractérisé en ce que** ledit composé amine A) est un mélange de composés amines A) tels que définis selon la revendication 1 ou 2 et/ou **en ce que** ledit composé acrylate B) est un mélange de composés B) tels que définis selon l'une des revendications 1 à 4 et/ou **en ce que** ledit composé acide C) est un mélange de composés C) tels que définis selon l'une des revendications 1, 4 à 7 et 9 à 11.

13. Oligomère selon l'une des revendications 1 à 12, **caractérisé en ce que** ledit oligomère précurseur P est défini selon la formule générale suivante (I) :

$$[[R_1\text{-}(R_2)N]_x\text{-}[R'_3\text{-}N(R'4)]_z\text{-}R_3\text{-}N(R_4)\text{-}CH_2CH_2\text{-}CO_2]_y\text{-}R_5\text{-}(CO_2\text{-}CH=CH_2)_{(n-y)} \qquad (I)$$

avec

$R_1$, $R_2$ étant alkyles en $C_1$ à $C_4$ d'une amine tertiaire différente d'un aminoacrylate ou $R_1$-N= et $R_2$-N=, étant

un groupement englobant une liaison aminoacrylate $R_6$-$O_2CCH_2CH_2N$- et dans ce cas avec $R_1$ ou $R_2$ identiques ou différents et correspondant à $R_6$-$O_2CCH_2CH_2$-, avec $R_6$ étant le résidu d'un composé monoacrylate, d'un monoacrylate de polyéther diol ou d'un monoacrylate de polyéther monoalcool,

$R_3$ : résidu dudit composé amine A), si porteur d'une fonction amine primaire et en option d'une fonction amine tertiaire, avec z = 0 et signifiant l'absence de fonction amine secondaire en présence de la fonction amine primaire,

$R'_3$ : est pour z = 0 et x = 1, une simple liaison entre $R_3$ et le groupement amine tertiaire « $R_1$-($R_2$)N- », ledit composé amine A portant une fonction amine primaire et une tertiaire ou

$R'_3$ : est un alkylène en $C_2$ à $C_6$ pour z = 1 et x = 1 avec ledit composé amine A) portant une fonction amine primaire, une fonction amine secondaire et une fonction amine tertiaire,

$R_4$ : pour z = 0 est un alkyle en $C_1$ à $C_4$ quand ledit composé amine A) porte une fonction amine secondaire et en option une fonction amine tertiaire,

sinon quand ledit composé amine A) porte une fonction amine primaire,

dans ce cas

$R_4$ : -[$CH_2CH_2$-$CO_2$]$_y$-$R_5$-($CO_2$-$CH$=$CH_2$)$_{(n-y)}$ ou

$R_4$ : au moins en partie est -$CH_2CH_2$-$CO_2$-$R_5$ quand il y a présence d'acrylate monofonctionnel de radical $R'_5$ en plus de l'acrylate multifonctionnel, avec $R'_5$ pouvant comprendre un groupement hydroxy libre,

$R'_4$ : -[$CH_2CH_2$-$CO_2$]$_y$-$R_5$-($CO_2$-$CH$=$CH_2$)$_{(n-y)}$ quand z = 1 signifiant présence simultanée d'une fonction amine primaire avec une secondaire,

$R_5$ : résidu dudit composé acrylate B) multifonctionnel, sélectionné parmi les résidus de polyéthers, de polyesters ou parmi les résidus de polyuréthanes à base de polyesters et de polyéthers polyols,

x = 1 si l'amine A) porte une fonction amine tertiaire et x = 0 s'il y absence d'une telle fonction amine tertiaire,

z = 1 si présence de fonction amine secondaire en même temps que la fonction amine primaire dans le composé amine A) et z = 0 si absence de fonction amine secondaire,

n : fonctionnalité initiale dudit acrylate B) allant de 1 à 6,

y : nombre de groupements aminoacrylates créés par réactions d'addition d'un N-H dudit composé amine A) sur un groupement acrylate dudit acrylate B), avec 'y' allant de 1 à 6, et n-y : de 0 à 3 représente le nombre de groupements acrylates résiduels dans ledit oligomère P.

**14.** Oligomère selon l'une des revendications 1 à 13, **caractérisé en ce que** ledit composé amine A) est un composé amine selon A1) tel que défini selon la revendication 1 ou 2 et que ledit composé acrylate B) comprend au moins un composé acrylate multifonctionnel selon B1) et au moins un composé acrylate monofonctionnel selon B2).

**15.** Oligomère selon l'une des revendications 1 à 13, **caractérisé en ce que** ledit composé amine A) est un composé amine selon A2) tel que défini selon la revendication 1 ou 2 et ledit composé acrylate B) est un composé acrylate multifonctionnel selon B1) sélectionné parmi les esters partiels ou complets par l'acide acrylique d'un polyéther polyol ou d'un polyol dérivé dudit polyéther ou parmi les uréthanes acrylates à partir d'un polyéther polyol ou parmi les époxy acrylates à partir d'un glycidyl polyéther.

**16.** Oligomère selon l'une des revendications 1 à 15, **caractérisé en ce que** ledit oligomère porte comme insaturation éthylénique des groupements acrylates avec une fonctionnalité en groupements acrylates, y compris acrylate d'ammonium si l'acide acrylique est utilisé comme composé acide carboxylique C), allant de 1 à 6.

**17.** Oligomère selon l'une des revendications 1 à 16, **caractérisé en ce qu'**il a un taux de fonction amine tertiaire salifiée allant de 0,1 à 25 mEq par g dudit oligomère.

**18.** Oligomère précurseur de l'oligomère tel que défini selon l'une des revendications 1 à 13, **caractérisé en ce qu'**il comprend ou est, un oligomère selon la formule (I) telle que définie selon la revendication 13.

**19.** Solution d'oligomère dans un diluant réactif, **caractérisée en ce qu'**elle comprend l'oligomère tel que défini selon l'une des revendications 1 à 18 et au moins un diluant réactif D) sélectionné parmi les mono (méth)acrylates et/ou les (méth)acrylates multifonctionnels.

**20.** Procédé de préparation d'un oligomère tel que défini selon l'une des revendications 1 à 18, **caractérisé en ce qu'**il comprend les étapes successives suivantes :

i) préparation dudit oligomère précurseur P par l'addition d'au moins un composé amine A), selon A1) et/ou selon A2) tel que défini selon la revendication 1 ou 2, sur B) un composé acrylate parmi :

## EP 3 359 607 B1

- B1) au moins un acrylate multifonctionnel ayant une fonctionnalité allant de 2 à 6 quand ledit composé amine A) est un composé selon A2) tel que défini selon la revendication 1 ou 2 ou
- un mélange d'au moins un acrylate multifonctionnel selon B1) comme défini ci-dessus et d'au moins un acrylate monofonctionnel selon B2), quand ledit composé amine A) est un composé amine selon A1), tel que défini selon la revendication 1 ou 2, ceci pour bloquer par l'aminoacrylate formé un des deux groupements -NH de ladite fonction amine primaire dudit composé amine selon A1),

avec les rapports molaires suivants entre groupements :

(acrylate) / (-NH) > 1 de sorte qu'au moins un groupement acrylate résiduel soit porté par le produit d'addition i), qui est ledit oligomère précurseur P

et dans le cas où ledit composé amine A) est selon A1) et ledit acrylate B) est un mélange d'acrylate multifonctionnel selon B1) et d'acrylate monofonctionnel selon B2) avec un rapport molaire B2 / A1 = 1 / 1

ii) salification dudit oligomère précurseur P qui est le produit de l'étape d'addition i), sous forme de sel de carboxylate d'ammonium par ledit composé acide carboxylique C), tel que défini selon l'une des revendications 1, 4 à 7 et 9 à 11, avec un rapport molaire entre groupements carboxy et fonctions amines tertiaires (=N-), y compris amines tertiaires parmi groupements aminoacrylates, (carboxy) / (amine tertiaire) allant de 0,10 à 1,00,.

21. Composition polymérisable, **caractérisée en ce qu'**elle comprend comme liant au moins un oligomère tel que défini selon l'une des revendications 1 à 18 ou obtenu par un procédé tel que défini selon la revendication 20 ou une solution telle que définie selon la revendication 19.

22. Composition selon la revendication 21, **caractérisée en ce qu'**elle est polymérisable par :

- voie de rayonnement parmi UV, LED, laser, faisceau d'électrons,
- voie thermique ou de peroxyde ou d'hydroperoxyde en présence d'un accélérateur,
- voie duale combinant au moins deux des voies précitées.

23. Utilisation d'une composition selon l'une des revendications 21 ou 22, **caractérisée en ce qu'**il s'agit d'utilisation pour matériau à fonction provisoire parmi revêtement, ou parmi hydrogel ou pour matériau support pour objet d'impression en 3D couche par couche.

24. Utilisation d'un oligomère tel que défini selon l'une des revendications 1 à 18 ou d'une solution telle que définie selon la revendication 19 ou de l'oligomère obtenu par un procédé tel que défini selon la revendication 20, en tant que liant polymérisable, en option hydrosoluble, dans des compositions polymérisables.

25. Utilisation selon la revendication 24, **caractérisée en ce qu'**elle s'applique à un matériau à fonction provisoire, éliminable par lavage à l'eau seule ou par une eau saline ou par une autre solution aqueuse ayant un pH > 7choisi parmi les revêtements, hydrogels ou un matériau support pour objet d'impression en 3D couche par couche.

26. Utilisation selon la revendication 24 ou 25, **caractérisée en ce qu'**elle s'applique à l'impression des objets 3D par polymérisation qui est une réticulation sous rayonnement couche par couche d'une composition comprenant ledit oligomère ou ladite solution d'oligomère comme matériau à fonction provisoire de support ou de consolidation ou de moulage dudit objet 3D final, obtenu après élimination dudit matériau à fonction provisoire par lavage à l'eau ou par une solution saline ou par une autre solution aqueuse.

27. Matériau polymère, **caractérisé en ce qu'**il résulte de l'utilisation comme liant polymérisable, d'au moins un oligomère tel que défini selon l'une des revendications 1 à 18 ou d'un oligomère obtenu par le procédé tel que défini selon la revendication 20, d'une solution d'oligomère telle que définie selon la revendication 19 ou de la polymérisation, d'une composition polymérisable, telle que définie selon l'une des revendications 21 à 24.

**Patentansprüche**

1. Polymerisierbares Oligomer, **dadurch gekennzeichnet, dass** es mindestens eine ethylenische Ungesättigtheit und mindestens eine ionische Ammoniumcarboxylat-Bindung trägt und dass es in seiner Struktur oder in seiner Zusammensetzung

P) mindestens ein Vorläufer-Oligomer mit mindestens einer tertiären Aminfunktion in Form einer Aminoacrylat-Gruppe aus der Addition von A) mindestens einer Aminverbindung gemäß A1) mit mindestens einer primären Aminfunktion (-NH$_2$) und/oder sekundären Aminfunktion (-NH) und gegebenenfalls mindestens einer tertiären Aminfunktion und/oder gemäß A2) mit mindestens einer sekundären Aminfunktion (-NH) und gegebenenfalls mindestens einer tertiären Aminfunktion an B) mindestens eine hydrophile Acrylatverbindung somit unter Bildung der mindestens einen Aminoacrylat-Gruppe und

C) mindestens eine Carbonsäureverbindung, die über mindestens eine ionische Ammoniumcarboxylat-Bindung mit mindestens einer der Aminoacrylat-Gruppen und gegebenenfalls mindestens einer der tertiären Aminfunktionen der Aminverbindung A) an das Vorläufer-Oligomer P gebunden ist,

umfasst.

2. Oligomer nach Anspruch 1, **dadurch gekennzeichnet, dass** die Aminverbindung A) aus

A1) einer Aminverbindung mit mindestens einer primären Aminfunktion und gegebenenfalls mindestens einer tertiären Aminfunktion und/oder mindestens einer sekundären Aminfunktion und/oder

A2) einer Aminverbindung mit mindestens einer sekundären Aminfunktion und gegebenenfalls mindestens einer tertiären Aminfunktion ausgewählt ist und dass es sich bei der Acrylatverbindung B) um mindestens eine multifunktionelle Acrylatverbindung gemäß B1) und/oder eine monofunktionelle Acrylatverbindung gemäß B2) handelt und es sich insbesondere für den Fall, dass A) eine Aminverbindung gemäß A1) ist oder umfasst, bei der Acrylatverbindung B) um eine Mischung von mindestens einem multifunktionellen Acrylat gemäß B1) und mindestens einem monofunktionellen Acrylat gemäß B2) handelt.

3. Oligomer nach Anspruch 2, **dadurch gekennzeichnet, dass** es sich bei der hydrophilen Acrylatverbindung B) um eine Mischung von multifunktioneller Acrylatverbindung gemäß B1) und monofunktioneller Acrylatverbindung gemäß B2) handelt.

4. Oligomer nach einem der Ansprüche 1 bis 3, **dadurch gekennzeichnet, dass** die Aminoacrylat-Gruppe mindestens eine ethylenische Ungesättigtheit oder keine ethylenische Ungesättigtheit tragen kann und in letzterem Fall die Säureverbindung C) ethylenisch ungesättigt ist.

5. Oligomer nach einem der Ansprüche 1 bis 4, **dadurch gekennzeichnet, dass** es sich bei der Säureverbindung C) um eine Monosäure gemäß C1), die ethylenisch ungesättigt gemäß C11) ist, handelt und das Oligomer mindestens zwei ionische Ammoniumcarboxylat-Bindungen trägt.

6. Oligomer nach einem der Ansprüche 1 bis 5, **dadurch gekennzeichnet, dass**:

- dann, wenn es sich bei der Carbonsäureverbindung C) gemäß C1) um eine Monosäure handelt:
es sich bei C1) um eine ethylenisch ungesättigte Monosäure gemäß C11) handelt und mindestens eine ethylenische Ungesättigtheit des Oligomers von mindestens der Monosäure gemäß C11) durch Ammoniumcarboxylat-Bindung mit mindestens einer der tertiären Aminfunktionen der Aminoacrylat-Gruppen oder gegebenenfalls der tertiären Aminfunktionen der Aminverbindung A) getragen wird und gegebenenfalls mindestens eine andere ethylenische Ungesättigtheit des Oligomers von mindestens einer Aminoacrylat-Gruppe in Form einer Aminoacrylat-Acrylatendgruppe des Vorläufer-Oligomers P getragen wird oder es sich bei C1) um eine gesättigte Monosäure gemäß C12) handelt und mindestens eine ethylenische Ungesättigtheit des Oligomers von mindestens einer Aminoacrylat-Gruppe in Form einer Aminoacrylat-Acrylatendgruppe des Vorläufer-Oligomers P getragen wird, und dann, wenn es sich bei der Carbonsäureverbindung C) gemäß C2) um eine Polycarbonsäure handelt:

es sich bei C2) um eine ethylenisch ungesättigte Polysäure gemäß C21) handelt und mindestens eine ethylenische Ungesättigtheit des Oligomers von mindestens der Polysäure gemäß C21) getragen wird und gegebenenfalls außerdem mindestens eine ethylenische Ungesättigtheit von mindestens einer Aminoacrylat-Gruppe in Form von Aminoacrylat-Acrylatendgruppen des Vorläufer-Oligomers P getragen wird oder es sich bei C2) um eine gesättigte Polysäure gemäß C22) handelt und das Vorläufer-Oligomer P mindestens eine Aminoacrylat-Acrylatendgruppe trägt und das resultierende Oligomer mindestens zwei ethylenische Acrylat-Ungesättigtheiten trägt.

7. Oligomer nach Anspruch 6, **dadurch gekennzeichnet, dass** es sich bei der Carbonsäureverbindung C) um eine ungesättigte Disäure gemäß C21) oder eine gesättigte Disäure gemäß C22) handelt, die über zwei ionische Carboxylat-Bindungen zwei Moleküle des Vorläufer-Oligomers P durch Versalzung einer der tertiären Aminfunktionen der gebildeten Aminoacrylat-Gruppen oder gegebenenfalls der tertiären Aminfunktionen der Aminverbindung A) in Ammoniumcarboxylatsalzform an jedem der Moleküle des Oligomers P verknüpft oder es sich bei der Carbonsäureverbindung C) um eine gemäß C11) ethylenisch ungesättigte Monosäure gemäß C1) mit einer polymerisierbaren ethylenischen Ungesättigtheit handelt und die Monosäure gemäß C11) mindestens zwei der tertiären Aminfunktionen in Ammoniumcarboxylatform versalzt.

8. Oligomer nach einem der Ansprüche 1 bis 7, **dadurch gekennzeichnet, dass** das Vorläufer-Oligomer P in seiner Struktur hydrophile Kettensegmente umfasst, die aus Polyethern oder aus Polyestern oder aus Polyurethanen auf Basis von Oligoetherpolyolen oder ethoxylierten Polyolen ausgewählt sind, wobei die Segmente eine zahlenmittlere Molmasse Mn von weniger als 2000 aufweisen.

9. Oligomer nach einem der Ansprüche 1 bis 8, **dadurch gekennzeichnet, dass** es sich bei der Carbonsäureverbindung C) gemäß C11) um eine ethylenisch ungesättigte Monosäure handelt, die aus Acryl- oder Methacrylsäure, Crotonsäure (trans-2-Buten-säure) oder β-Carboxyethylacrylat (β-CEA) oder Mischungen davon ausgewählt ist.

10. Oligomer nach einem der Ansprüche 1 bis 8, **dadurch gekennzeichnet, dass** es sich bei der Carbonsäureverbindung C) gemäß C22) um eine gesättigte Dicarbonsäure handelt, die aus Bernsteinsäure, Malonsäure, Äpfelsäure, Glutarsäure ($C_5$: trägt 5 Kohlenstoffatome), Adipinsäure ($C_6$), Pimelinsäure ($C_7$) oder Säurediestern von oben aufgeführten Disäuren mit einem $C_2$- bis $C_4$-Alkandiol oder mit einem Di-, Tri- oder Tetraethylenglykol oder Disäuren aus Fettsäuredimeren und/oder -trimeren oder Mischungen von zwei oder drei davon ausgewählt ist.

11. Oligomer nach einem der Ansprüche 1 bis 8, **dadurch gekennzeichnet, dass** es sich bei der Carbonsäureverbindung C) gemäß C21) um eine ungesättigte Dicarbonsäure handelt, die aus Itaconsäure, Maleinsäure, Fumarsäure, Tetrahydrophthalsäure (Cyclohexendisäure) oder Säurediestern der oben aufgeführten Disäuren mit einem $C_2$- bis $C_4$-Alkan-diol oder mit einem Di-, Tri- oder Tetraethylenglykol oder nicht hydrierten Fettsäuredimeren und/oder -trimeren mit $C_{36}$-Dimeren und $C_{54}$-Trimeren oder Mischungen davon ausgewählt ist.

12. Oligomer nach einem der Ansprüche 1 bis 8, **dadurch gekennzeichnet, dass** es sich bei der Aminverbindung A) um eine Mischung von Aminverbindungen A) gemäß Anspruch 1 oder 2 handelt und/oder es sich bei der Acrylatverbindung B) um eine Mischung von Verbindungen B) gemäß einem der Ansprüche 1 bis 4 handelt und/oder es sich bei der Säureverbindung C) um eine Mischung von Verbindungen C) gemäß einem der Ansprüche 1, 4 bis 7 und 9 bis 11 handelt.

13. Oligomer nach einem der Ansprüche 1 bis 12, **dadurch gekennzeichnet, dass** das Vorläufer-Oligomer P gemäß der folgenden allgemeinen Formel (I) definiert ist:

$$[[R_1\text{-}(R_2)N]_x\text{-}[R'_3\text{-}N(R'4)]\ _z\text{-}R_3\text{-}N(R_4)\text{-}CH_2CH_2\text{-}CO_2]_y\text{-}R_5\text{-}(CO_2\text{-}CH=CH_2)_{(n-y)} \qquad (I)$$

wobei

$R_1$ und $R_2$ für $C_1$- bis $C_4$-Alkylgruppen eines tertiären Amins, das von einem Aminoacrylat verschieden ist, stehen oder $R_1$-N= und $R_2$-N= für eine Gruppe, die eine Aminoacrylat-Bindung $R_6$-$O_2CCH_2CH_2N$- umfasst, stehen und in diesem Fall $R_1$ oder $R_2$ gleich oder verschieden sind und $R_6$-$O_2CCH_2CH_2$- entsprechen, wobei $R_6$ für den Rest einer Monoacrylat-Verbindung, eines Polyetherdiolmonoacrylats oder eines Polyethermonoalkoholmonoacrylats steht,
$R_3$ für einen Rest der Aminverbindung A) steht, wenn sie eine primäre Aminfunktion und gegebenenfalls eine tertiäre Aminfunktion trägt, wobei z = 0 ist und die Abwesenheit einer sekundären Aminfunktion in Gegenwart der primären Aminfunktion anzeigt,
$R'_3$ für z = 0 und x = 1 für eine Einfachbindung zwischen $R_3$ und der tertiären Aminogruppe "$R_1$-(R2)N-" steht, wobei die Aminverbindung A eine primäre Aminfunktion und eine tertiäre Aminfunktion trägt, oder
$R'_3$ für z = 1 und x = 1 für ein $C_2$- bis $C_6$-Alkylen steht, wobei die Aminverbindung A) eine primäre Aminfunktion, eine sekundäre Aminfunktion und eine tertiäre Aminfunktion trägt,
$R_4$ für z = 0 für ein $C_1$- bis $C_4$-Alkyl steht, wenn die Aminverbindung A) eine sekundäre Aminfunktion und gegebenenfalls eine tertiäre Aminfunktion trägt,
außer dann, wenn die Aminverbindung A) eine primäre Aminfunktion trägt,
wobei in diesem Fall
$R_4$ für -$[CH_2CH_2\text{-}CO_2]_y$-$R_5$-$(CO_2\text{-}CH=CH_2)_{(n-y)}$ steht oder $R_4$ mindestens teilweise für -$CH_2CH_2\text{-}CO_2$-$R'_5$ steht,

wenn neben dem multifunktionellen Acrylat ein monofunktionelles Acrylat mit Rest $R'_5$ vorliegt, wobei $R'_5$ eine freie Hydroxygruppe umfassen kann,

$R'_4$ für $-[CH_2CH_2\text{-}CO_2]_y\text{-}R_5\text{-}(CO_2\text{-}CH=CH_2)_{(n-y)}$ steht, wenn z = 1 die gleichzeitige Gegenwart einer primären Aminfunktion mit einer sekundären Aminfunktion anzeigt,

$R_5$ für einen Rest der multifunktionellen Acrylatverbindung B) steht, der aus Polyetherresten, Polyesterresten oder Resten von Polyurethanen auf Basis von Polyestern und Polyetherpolyolen ausgewählt ist,

x = 1, wenn das Amin A) eine tertiäre Aminfunktion trägt, und x = 0, wenn eine derartige tertiäre Aminfunktion fehlt,

z = 1, wenn eine sekundäre Aminfunktion gleichzeitig mit der primären Aminfunktion in der Aminverbindung A) vorliegt, und z = 0, wenn eine sekundäre Aminfunktion fehlt,

n für die Anfangsfunktionalität des Acrylats B) im Bereich von 1 bis 6 steht,

y für die Zahl der durch Additionsreaktionen eines N-H der Aminverbindung A) an eine Acrylatgruppe des Acrylats B) erzeugten Aminoacrylat-Gruppen steht, wobei "y" im Bereich von 1 bis 6 liegt und

n-y für 0 bis 3 steht und die Zahl der restlichen Acrylatgruppen in dem Oligomer P angibt.

14. Oligomer nach einem der Ansprüche 1 bis 13, **dadurch gekennzeichnet, dass** es sich bei der Aminverbindung A) um eine Aminverbindung gemäß A1) gemäß Anspruch 1 oder 2 handelt und die Acrylatverbindung B) mindestens eine multifunktionelle Acrylatverbindung gemäß B1) und mindestens eine monofunktionelle Acrylatverbindung gemäß B2) umfasst.

15. Oligomer nach einem der Ansprüche 1 bis 13, **dadurch gekennzeichnet, dass** es sich bei der Aminverbindung A) um eine Aminverbindung gemäß A2) gemäß Anspruch 1 oder 2 handelt und es sich bei der Acrylatverbindung B) um eine multifunktionelle Acrylatverbindung gemäß B1) handelt, die aus Partial- oder Vollestern von Acrylsäure mit einem Polyetherpolyol oder einem Polyol, das sich von dem Polyether ableitet, oder aus Urethanacrylaten ausgehend von einem Polyetherpolyol oder aus Epoxyacrylaten ausgehend von einem Glycidylpolyether ausgewählt ist.

16. Oligomer nach einem der Ansprüche 1 bis 15, **dadurch gekennzeichnet, dass** das Oligomer als ethylenische Ungesättigtheit Acrylatgruppen mit einer Funktionalität von Acrylatgruppen einschließlich Ammoniumacrylat, wenn Acrylsäure als Carbonsäureverbindung C) verwendet wird, im Bereich von 1 bis 6 trägt.

17. Oligomer nach einem der Ansprüche 1 bis 16, **dadurch gekennzeichnet, dass** es einen Gehalt an versalzter tertiärer Aminfunktion im Bereich von 0,1 bis 25 mEq pro g des Oligomers aufweist.

18. Vorläufer-Oligomer für das Oligomer gemäß einem der Ansprüche 1 bis 13, **dadurch gekennzeichnet, dass** es ein Oligomer gemäß Formel (I) gemäß Anspruch 13 umfasst oder ist.

19. Lösung von Oligomer in einem reaktiven Verdünnungsmittel, **dadurch gekennzeichnet, dass** sie das Oligomer gemäß einem der Ansprüche 1 bis 18 und mindestens ein reaktives Verdünnungsmittel D), das aus Mono(meth)acrylaten und/oder multifunktionellen (Meth)acrylaten ausgewählt ist, umfasst.

20. Verfahren zur Herstellung eines Oligomers gemäß einem der Ansprüche 1 bis 18, **dadurch gekennzeichnet, dass** es die folgenden aufeinanderfolgenden Schritte umfasst:

i) Herstellung des Vorläufer-Oligomers P durch Addition mindestens einer Aminverbindung A) gemäß A1) und/oder gemäß A2) gemäß Anspruch 1 oder 2 an B) eine Acrylatverbindung aus:

- B1) mindestens einem multifunktionellen Acrylat mit einer Funktionalität im Bereich von 2 bis 6, wenn es sich bei der Aminverbindung A) um eine Verbindung gemäß A2) gemäß Anspruch 1 oder 2 handelt, oder
- einer Mischung von mindestens einem multifunktionellen Acrylat gemäß B1) gemäß obigen Angaben und mindestens einem monofunktionellen Acrylat gemäß B2), wenn es sich bei der Aminverbindung A) um eine Aminverbindung gemäß A1) gemäß Anspruch 1 oder 2 handelt, und zwar zur Blockierung einer der beiden NH-Gruppen der primären Aminfunktion der Aminverbindung gemäß A1) durch das gebildete Aminoacrylat, mit den folgenden Molverhältnissen zwischen Gruppen:

(Acrylat) / (-NH) > 1, so dass das Additionsprodukt i), bei dem es sich um das Vorläufer-Oligomer P handelt, mindestens eine verbliebene Acrylatgruppe trägt,
und in dem Fall, dass die Aminverbindung A) gemäß A1) ist und es sich bei dem Acrylat B) um eine Mischung von multifunktionellem Acrylat gemäß B1) und monofunktionellem Acrylat gemäß B2) handelt, mit einem Molverhältnis B2 / A1 = 1 / 1,

ii) Versalzung des Vorläufer-Oligomers P, bei dem es sich um das Produkt des Additionsschritts i) handelt, in Ammoniumcarboxylatsalzform durch die Carbonsäureverbindung C) gemäß einem der Ansprüche 1, 4 bis 7 und 9 bis 11 mit einem Molverhältnis zwischen Carboxygruppen und tertiären Aminfunktionen (=N-), einschließlich tertiärer Amine unter Aminoacrylat-Gruppen, (Carboxy) / (tertiäres Amin) im Bereich von 0,10 bis 1,00.

21. Polymerisierbare Zusammensetzung, **dadurch gekennzeichnet, dass** sie als Bindemittel mindestens ein Oligomer gemäß einem der Ansprüche 1 bis 18 oder ein durch ein Verfahren gemäß Anspruch 20 erhaltenes Oligomer oder eine Lösung gemäß Anspruch 19 umfasst.

22. Zusammensetzung nach Anspruch 21, **dadurch gekennzeichnet, dass** sie

   - durch Strahlung, darunter UV, LED, Laser, Elektronenstrahlung,
   - thermisch oder durch Peroxid oder Hydroperoxid in Gegenwart eines Beschleunigers,
   - auf dualem Wege unter Kombination von mindestens zwei der oben genannten Wege polymerisierbar ist.

23. Verwendung einer Zusammensetzung nach einem der Ansprüche 21 oder 22, **dadurch gekennzeichnet, dass** es sich um die Verwendung als Material mit provisorischer Funktion unter Beschichtungen oder Hydrogelen oder als Trägermaterial für einen schichtweise durch 3D-Druck aufgebauten Gegenstand handelt.

24. Verwendung eines Oligomers gemäß einem der Ansprüche 1 bis 18 oder einer Lösung gemäß Anspruch 19 oder des durch ein Verfahren gemäß Anspruch 20 erhaltenen Oligomers als gegebenenfalls wasserlösliches polymerisierbares Bindemittel in polymerisierbaren Zusammensetzungen.

25. Verwendung nach Anspruch 24, **dadurch gekennzeichnet, dass** sie auf ein Material mit provisorischer Funktion, das durch Waschen mit Wasser alleine oder mit Salzwasser oder mit einer anderen wässrigen Lösung mit einem pH-Wert > 7 entfernbar ist, das aus Beschichtungen, Hydrogelen oder einem Trägermaterial für einen schichtweise durch 3D-Druck aufgebauten Gegenstand ausgewählt ist, zutrifft.

26. Verwendung nach Anspruch 24 oder 25, **dadurch gekennzeichnet, dass** sie auf den Druck von 3D-Gegenständen durch Polymerisation, wobei es sich um eine schichtweise Vernetzung einer Zusammensetzung, die das Oligomer oder die Oligomerlösung umfasst, unter Strahlung handelt, als Material mit provisorischer Funktion zum Tragen oder zur Konsolidierung oder Formung des nach Entfernung des Materials mit provisorischer Funktion durch Waschen mit Wasser oder mit einer Salzlösung oder mit einer anderen wässrigen Lösung erhaltenen fertigen 3D-Gegenstands zutrifft.

27. Polymermaterial, **dadurch gekennzeichnet, dass** es sich aus der Verwendung mindestens eines Oligomers gemäß einem der Ansprüche 1 bis 18 oder eines durch das Verfahren gemäß Anspruch 20 erhaltenen Oligomers, einer Oligomerlösung gemäß Anspruch 19 oder der Polymerisation einer polymerisierbaren Zusammensetzung gemäß einem der Ansprüche 21 bis 24 als polymerisierbares Bindemittel ergibt.

**Claims**

1. Polymerizable oligomer, **characterized in that** it bears at least one ethylenic unsaturation and at least one ammonium carboxylate ionic bond and **in that** it comprises in its structure or in its composition:

   P) at least one precursor oligomer bearing at least one tertiary amine function in the form of an aminoacrylate group, resulting from the addition of A) at least one amine compound according to A1) bearing at least one primary amine function ($-NH_2$) and/or secondary amine function (-NH) and optionally at least one tertiary amine function and/or according to A2) bearing at least one secondary amine function (-NH) and optionally at least one tertiary amine function, on B) at least one hydrophilic acrylate compound with as a result the formation of said at least one aminoacrylate group, and
   C) at least one carboxylic acid compound attached to said precursor oligomer P by at least one ammonium carboxylate ionic bond with at least one of said aminoacrylate groups and optionally at least one of said tertiary amine functions of said amine compound A).

2. Oligomer according to Claim 1, **characterized in that** said amine compound A) is selected from:

A1) an amine compound bearing at least one primary amine function, and optionally at least one tertiary amine function and/or at least one secondary amine function, and/or

A2) an amine compound bearing at least one secondary amine function, and optionally at least one tertiary amine function,

and **in that** said acrylate compound B) is at least one multifunctional acrylate compound according to B1) and/or monofunctional acrylate compound according to B2), and in particular for A) being or comprising an amine compound according to A1), said acrylate compound B) is a mixture of at least one multifunctional acrylate according to B1) and of at least one monofunctional acrylate according to B2).

3. Oligomer according to Claim 2, **characterized in that** said hydrophilic acrylate compound B) is a mixture of multifunctional acrylate compound according to B1) and of monofunctional acrylate compound according to B2).

4. Oligomer according to one of Claims 1 to 3, **characterized in that** said aminoacrylate group can bear at least one ethylenic unsaturation or no ethylenic unsaturation and, in the latter case, said acid compound C) is ethylenically unsaturated.

5. Oligomer according to one of Claims 1 to 4, **characterized in that** said acid compound C) is a monoacid according to C1) which is ethylenically unsaturated according to C11) and said oligomer bears at least two ammonium carboxylate ionic bonds.

6. Oligomer according to one of Claims 1 to 5, **characterized in that**:

   - when said carboxylic acid compound C) is according to C1) a monoacid, in this case:

      C1) is an ethylenically unsaturated monoacid according to C11) and at least one ethylenic unsaturation of said oligomer is borne by at least said monoacid according to C11) by ammonium carboxylate bonding with at least one of said tertiary amine functions among said aminoacrylate groups or optionally among the tertiary amine functions borne by said amine compound A) and optionally at least one other ethylenic unsaturation of said oligomer is borne by at least one aminoacrylate group in the form of an aminoacrylate-end acrylate group of said precursor oligomer P or

      C1) is a saturated monoacid according to C12) and at least one ethylenic unsaturation of said oligomer is borne by at least one aminoacrylate group in the form of an aminoacrylate-end acrylate group of said precursor oligomer P and

   - when said carboxylic acid compound C) is according to C2) a polycarboxylic acid and in this case:

      C2) is an ethylenically unsaturated polyacid according to C21) and at least one ethylenic unsaturation of said oligomer is borne by at least said polyacid according to C21) and optionally, in addition, at least one ethylenic unsaturation is borne by at least one aminoacrylate group in the form of an aminoacrylate-end acrylate group of said precursor oligomer P or

      C2) is a saturated polyacid according to C22) and said precursor oligomer P bears at least one aminoacrylate-end acrylate group and said resulting oligomer bears at least two acrylate ethylenic unsaturations.

7. Oligomer according to Claim 6, **characterized in that** said carboxylic acid compound C) is an unsaturated diacid according to C21) or a saturated diacid according to C22) linking, by two carboxylate ionic bonds, two molecules of said precursor oligomer P, by salification in ammonium carboxylate salt form of one of said tertiary amine functions among said aminoacrylate groups formed, or optionally among said tertiary amine functions of said amine compound A), on each of said molecules of said oligomer P or **in that** said carboxylic acid compound C) is a monoacid according to C1), which is ethylenically unsaturated according to C11) and has a polymerizable ethylenic unsaturation, and **in that** said monoacid according to C11) salifies at least two of said tertiary amine functions in ammonium carboxylate form.

8. Oligomer according to one of Claims 1 to 7, **characterized in that** said precursor oligomer P comprises, in its structure, hydrophilic chain segments selected from polyethers or from polyesters, or from polyurethanes based on oligoether polyols or on ethoxylated polyols, said segments having a number-average molecular weight Mn of less than 2000.

9. Oligomer according to one of Claims 1 to 8, **characterized in that** said carboxylic acid compound C) is according to C11) an ethylenically unsaturated monoacid and selected from: acrylic or methacrylic acid, crotonic acid (trans-2-butenoic acid) or $\beta$-carboxyethyl acrylate ($\beta$-CEA) or mixtures thereof.

10. Oligomer according to one of Claims 1 to 8, **characterized in that** said carboxylic acid compound C) is according to C22) a saturated dicarboxylic acid and selected from: succinic acid, malonic acid, malic acid, glutaric acid ($C_5$: bearing 5 carbon atoms), adipic acid ($C_6$), pimelic acid ($C_7$) or acid diesters of abovementioned diacids with a $C_2$ to $C_4$ alkanediol or with a di-, tri- or tetraethylene glycol or diacids among fatty acid dimers and/or trimers, or mixtures of two or of three thereof.

11. Oligomer according to one of Claims 1 to 8, **characterized in that** said carboxylic acid compound C) is according to C21) an unsaturated dicarboxylic acid and selected from: itaconic acid, maleic acid, fumaric acid, tetrahydrophthalic acid (cyclohexenedioic acid), or acid diesters of the abovementioned diacids with a $C_2$ to $C_4$ alkanediol or with a di-, tri- or tetraethylene glycol or non-hydrogenated fatty acid dimers and/or trimers with $C_{36}$ dimers and $C_{54}$ trimers, or mixtures thereof.

12. Oligomer according to one of Claims 1 to 8, **characterized in that** said amine compound A) is a mixture of amine compounds A) as defined in Claim 1 or 2 and/or **in that** said acrylate compound B) is a mixture of compounds B) as defined in one of Claims 1 to 4 and/or **in that** said acid compound C) is a mixture of compounds C) as defined in one of Claims 1, 4 to 7 and 9 to 11.

13. Oligomer according to one of Claims 1 to 12, **characterized in that** said precursor oligomer P is defined according to general formula (I) below:

$$[[R_1\text{-}(R_2)N]_x\text{-}[R'_3\text{-}N(R'4)]_z\text{-}R_3\text{-}N(R_4)\text{-}CH_2CH_2\text{-}CO_2]_y\text{-}R_5\text{-}(CO_2\text{-}CH=CH_2)_{(n-y)} \qquad (I)$$

with

$R_1$, $R_2$ being $C_1$ to $C_4$ alkyls of a different tertiary amine of an aminoacrylate or $R_1$-N= and $R_2$-N=, being a group encompassing an aminoacrylate bond $R_6$-$O_2CCH_2CH_2N$- and in this case with $R_1$ or $R_2$ identical or different and corresponding to $R_6$-$O_2CCH_2CH_2$-, with $R_6$ being the residue of a monoacrylate compound, a polyether diol monoacrylate or a polyether monoalcool monoacrylate,
$R_3$: residue of said amine compound A), if bearing a primary amine function and optionally a tertiary amine function, with z = 0 and meaning the absence of secondary amine function in the presence of the primary amine function,
$R'_3$: is, for z = 0 and x = 1, a single bond between $R_3$ and the tertiary amine group "$R_1$-$(R_2)N$-", said amine compound A bearing a primary amine function and a tertiary or
$R'_3$: is a $C_2$ to $C_6$ alkylene for z = 1 and x = 1 with said amine compound A) bearing a primary amine function, a secondary amine function and a tertiary amine function,
$R_4$: for z = 0, is a $C_1$ to $C_4$ alkyl when said amine compound A) bears a secondary amine function and optionally a tertiary amine function,
if not, when said amine compound A) bears a primary amine function,
in this case
$R_4$: -$[CH_2CH_2\text{-}CO_2]_y$-$R_5$-$(CO_2\text{-}CH=CH_2)_{(n-y)}$ or
$R_4$: at least one part is -$CH_2CH_2\text{-}CO_2$-$R'_5$ when a monofunctional acrylate of radical $R'_5$ is present in addition to the multifunctional acrylate, with $R'_5$ possibly comprising a free hydroxyl group,
$R'_4$: -$[CH_2CH_2\text{-}CO_2]_y$-$R_5$-$(CO_2\text{-}CH=CH_2)_{(n-y)}$ when z = 1 meaning the simultaneous presence of a primary amine function with a secondary,
$R_5$: residue of said multifunctional acrylate compound B), selected from residues of polyethers, of polyesters or from residues of polyurethanes based on polyesters and polyether polyols,
x = 1 if the amine A) bears a tertiary amine function and x = 0 if such a tertiary amine function is absent,
z = 1 if a secondary amine function is present at the same time as the primary amine function in the amine compound A) and z = 0 if a secondary amine function is absent,
n: initial functionality of said acrylate B) ranging from 1 to 6,
y: number of aminoacrylate groups created by addition reactions of an N-H of said amine compound A) on an acrylate group of said acrylate B), with 'y' ranging from 1 to 6, and
n-y: from 0 to 3 represents the number of residual acrylate groups in said oligomer P.

14. Oligomer according to one of Claims 1 to 13, **characterized in that** said amine compound A) is an amine compound according to A1) as defined in Claim 1 or 2 and that said acrylate compound B) comprises at least one multifunctional acrylate compound according to B1) and at least one monofunctional acrylate compound according to B2).

15. Oligomer according to one of Claims 1 to 13, **characterized in that** said amine compound A) is an amine compound according to A2) as defined in Claim 1 or 2 and said acrylate compound B) is a multifunctional acrylate compound according to B1) selected from partial or complete acrylic acid esters of a polyether polyol or of a polyol derived from said polyether, or from urethane acrylates from a polyether polyol, or from epoxy acrylates from a glycidyl polyether.

16. Oligomer according to one of Claims 1 to 15, **characterized in that** said oligomer bears, as ethylenic unsaturation, acrylate groups with a functionality with respect to acrylate groups, including ammonium acrylate if acrylic acid is used as carboxylic acid compound C), ranging from 1 to 6.

17. Oligomer according to one of Claims 1 to 16, **characterized in that** it has an amount of salified tertiary amine function ranging from 0.1 to 25 mEq per g of said oligomer.

18. Precursor oligomer of the oligomer as defined in one of Claims 1 to 13, **characterized in that** it comprises or is an oligomer according to formula (I) as defined in Claim 13.

19. Solution of oligomer in a reactive diluent, **characterized in that** it comprises the oligomer as defined in one of Claims 1 to 18 and at least one reactive diluent D) selected from mono(meth)acrylates and/or multifunctional (meth)acrylates.

20. Process for preparing an oligomer as defined in one of Claims 1 to 18, **characterized in that** it comprises the following successive steps:

   i) preparation of said precursor oligomer P by addition of at least one amine compound A), according to A1) and/or according to A2) as defined in Claim 1 or 2, on B) an acrylate compound from:

   - B1) at least one multifunctional acrylate having a functionality ranging from 2 to 6 when said amine compound A) is a compound according to A2) as defined in Claim 1 or 2 or
   - a mixture of at least one multifunctional acrylate according to B1) as defined above and of at least one monofunctional acrylate according to B2), when said amine compound A) is an amine compound according to A1), as defined in Claim 1 or 2, this being for blocking, by the aminoacrylate formed, one of the two -NH groups of said primary amine function of said amine compound according to A1),
   with the following molar ratios between groups:

   (acrylate) / (-NH) > 1 so that at least one residual acrylate group is borne by the addition product i), which is said precursor oligomer P,
   and in the case where said amine compound A) is according to A1) and said acrylate B) is a mixture of multifunctional acrylate according to B1) and of monofunctional acrylate according to B2) with a B2 /A1 molar ratio = 1 / 1,

   ii) salification of said precursor oligomer P which is the product of the addition step i), in ammonium carboxylate salt form, by said carboxylic acid compound C), as defined in one of Claims 1, 4 to 7 and 9 to 11, with a (carboxy)/(tertiary amine) molar ratio between carboxy groups and tertiary amine functions (=N-), including tertiary amines among aminoacrylate groups, ranging from 0.10 to 1.00.

21. Polymerizable composition, **characterized in that** it comprises, as binder, at least one oligomer as defined in one of Claims 1 to 18 or obtained by means of a process as defined in Claim 20, or a solution as defined in Claim 19.

22. Composition according to Claim 21, **characterized in that** it is polymerizable:

   - by UV, LED, laser or electron beam radiation,
   - thermally or by peroxide or by hydroperoxide in the presence of an accelerator,
   - by a dual route combining at least two of the abovementioned routes.

23. Use of a composition according to either of Claims 21 and 22, **characterized in that** it is a use for a temporary-use material among a coating, or among a hydrogel or for a support material for a layer-by-layer 3D printing object.

24. Use of an oligomer as defined in one of Claims 1 to 18 or of a solution as defined in Claim 19 or of the oligomer obtained by means of a process as defined in Claim 20, as a polymerizable binder, which is optionally water-soluble, in polymerizable compositions.

25. Use according to Claim 24, **characterized in that** it applies to a temporary-use material which can be removed by washing with water alone or with salt water or with another aqueous solution, having a pH > 7, chosen from coatings, hydrogels or a support material for a layer-by-layer 3D printing object.

26. Use according to Claim 24 or 25, **characterized in that** it applies to the printing of 3D objects by polymerization which is a crosslinking under radiation layer by layer of a composition comprising said oligomer or said solution of oligomer, as temporary-use material for support or consolidation or molding of said final 3D object, obtained after removal of said temporary-use material by washing with water or with a saline solution or with another aqueous solution.

27. Polymer material, **characterized in that** it results from the use, as a polymerizable binder, of at least one oligomer as defined in one of Claims 1 to 18 or of an oligomer obtained by means of the process as defined in Claim 20, of a solution of oligomer as defined in Claim 19, or from the polymerization of a polymerizable composition as defined in one of Claims 21 to 24.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20130234370 A
- US 20130337277 A
- US 20120178845 A
- CA 2239439
- CA 2239310
- US 5792827 A
- US 20120157351 A